# EUROPÄISCHE PATENTSCHRIFT

(11) **EP 4 126 078 B1**
(45) Veröffentlichungstag und Bekanntmachung des Hinweises auf die Patenterteilung: **28.10.2026**
(21) Anmeldenummer: 21713382.6
(22) Anmeldetag: 18.03.2021
(51) Int. Cl.: A61L 2/14

(54) **VORRICHTUNG ZUR DESINFEKTION VON GEGENSTÄNDEN ODER FESTSTOFFEN, VORZUGSWEISE VON SCHUTZAUSRÜSTUNGSTEILEN, UND DEREN VERWENDUNG**
DEVICE FOR DISINFECTING ITEMS OR SOLID MATTER, PREFERABLY PIECES OF PROTECTIVE EQUIPMENT, AND THE USE OF SAID DEVICE
DISPOSITIF DE DÉSINFECTION D'OBJETS OU DE MATIÈRES SOLIDES, DE PRÉFÉRENCE DES ÉLÉMENTS D'ÉQUIPEMENT DE PROTECTION ET UTILISATION DUDIT DISPOSITIF

(30) Priorität: 23.03.2020 DE 102020107981
(43) Veröffentlichungstag der Anmeldung: 08.02.2023
(73) Patentinhaber: Plasmatreat GmbH, 33803 Steinhagen (DE)
(72) Erfinder: SCHMITT-JOHN, Thomas, 33803 Steinhagen (DE); HASSE, Daniel, 33104 Paderborn (DE); KLÄSENER, Roland Heinrich, 33758 Schloß Holte Stuckenbrock (DE); BUSKE, Christian, 33619 Bielefeld (DE)
(74) Vertreter: Cohausz & Florack
(86) Internationale Anmeldenummer: PCT/EP2021/056920
(87) Internationale Veröffentlichungsnummer: WO 2021/191040

(56) Entgegenhaltungen:
- WO-A1-2019/103444
- KR-A- 20100 055 073
- KR-A- 20130 111 808
- KR-B1- 100 873 412
- KR-B1- 101 873 689

## Beschreibung

Die vorliegende Erfindung betrifft eine Vorrichtung zur Desinfektion, insbesondere zur Sterilisation, von Gegenständen oder Feststoffen, vorzugsweise von Schutzausrüstungsteilen, insbesondere von Schutzmasken oder Schutzbekleidung.

Zum Schutz vor Krankheitserregern wie zum Beispiel Bakterien, Viren oder Sporen werden insbesondere Schutzmasken oder Schutzbekleidung wie zum Beispiel Schutzanzüge eingesetzt. Derartige Schutzausrüstungsteile können typischerweise nur über einen begrenzten Zeitraum verwendet werden, bevor sie gewechselt werden müssen. Zum Beispiel müssen Schutzmasken, je nach Art, täglich, nach einigen Stunden, stündlich oder sogar in noch kürzeren Abständen gewechselt werden. Nach der Verwendung müssen die Schutzausrüstungsteile aufwändig desinfiziert oder im Fall von Einmal-Schutzausrüstungsteilen direkt entsorgt werden.

In Zeiten erhöhten Bedarfs oder bei Nachschubknappheit kann es zu einer Unterversorgung mit Schutzausrüstungsteilen kommen.

Es besteht ein Bedürfnis danach, Schutzausrüstungsteile schnell und einfach zu desinfizieren, um diese schneller wieder für den Einsatz zur Verfügung stellen zu können. Weiterhin besteht ein Bedürfnis, Schutzausrüstungsteile, die sonst nur für die Einmalverwendung in Frage kommen, länger verwenden zu können bzw. wiederverwenden zu können. Eine bekannte Desinfektionsanlage ist im Dokument KR101873689B1 veröffentlicht.

Hiervon ausgehend stellt sich vorliegend die Aufgabe, eine Lösung zur zumindest teilweisen Befriedigung mindestens eines der zuvor genannten Bedürfnisse vorzuschlagen.

Diese Aufgabe wird erfindungsgemäß gelöst durch eine Vorrichtung gemäß Anspruch 1.

Mit einer solchen Vorrichtung lassen sich Gegenstände oder Feststoffe schnell, einfach und zuverlässig desinfizieren. Bei den Gegenständen oder Feststoffen kann es sich insbesondere um ein oder mehrere Güter handeln. Insbesondere kann es sich bei den Gegenständen um ein oder mehrere Stücke eines oder mehrerer Güter handeln.

Mit der zuvor beschriebenen Vorrichtung lassen sich insbesondere Schutzausrüstungsteile schnell, einfach und zuverlässig desinfizieren, insbesondere sterilisieren, um sie für einen neuen Einsatz bereitzustellen. Weiterhin können mit einer solchen Vorrichtung zum Beispiel auch Schutzausrüstungsteile wie zum Beispiel Atemschutzmasken desinfiziert werden, die sonst nur für den Einmalgebrauch vorgesehen sind. Auf diese Weise können die Verwendbarkeitsdauer der Schutzausrüstungsteile verlängert bzw. die Schutzausrüstungsteile soweit zulässig wiederverwendet werden.

Neben der Desinfektion von Schutzausrüstungsteilen kommt die Vorrichtung weiterhin zur Desinfektion anderer Güter wie Pulver, Saatgut oder Lebensmittel, insbesondere Gemüse, Obst, Salat, Nüsse wie zum Beispiel Haselnüsse, Mandeln, Hülsenfrüchte oder Gewürze wie zum Beispiel Pfeffer, in Betracht.

Die Vorrichtung weist eine Behandlungskammer auf. Die Behandlungskammer ist vorzugsweise bis auf für den Betrieb erforderliche Ein- und Auslässe vollständig verschließbar, so dass im Betrieb keine reaktiven Spezies unkontrolliert aus der Behandlungskammer austreten können. Die Behandlungskammer ist insbesondere so dimensioniert, dass sie ein oder mehrere Gegenstände oder Feststoffe, bevorzugt ein, vorzugsweise mehrere Schutzausrüstungsteile, insbesondere ein oder mehrere Atemschutzmasken und/oder Schutzbekleidungsteile aufnehmen kann.

Die Vorrichtung weist weiter eine verschließbare Schleuse auf, durch die Gegenstände oder Feststoffe, vorzugsweise Schutzausrüstungsteile, in die Behandlungskammer eingebracht und/oder aus der Behandlungskammer entnommen werden können. Bei der Schleuse kann es sich insbesondere um eine in einer Seitenwand der Behandlungskammer vorgesehene Tür handeln, die im geöffneten Zustand den Zugang zur Behandlungskammer ermöglicht, so dass Gegenstände oder Feststoffe, vorzugsweise Schutzausrüstungsteile, in die Behandlungskammer eingebracht und/oder aus der Behandlungskammer entnommen werden können, und die im geschlossenen Zustand die Behandlungskammer verschließt.

Die Vorrichtung weist weiter eine Erzeugungseinheit zur Erzeugung reaktiver Spezies in einem Gasstrom auf. Die Erzeugungseinheit ist demnach insbesondere dazu eingerichtet, reaktive Spezies in einem Gasstrom zu erzeugen. Zu diesem Zweck umfasst die Erzeugungseinheit Entladungsmittel, die dazu eingerichtet sind, eine elektrische Entladung in dem Gasstrom zu erzeugen. Die Entladungsmittel können insbesondere Elektroden umfassen, an die eine hochfrequente Hochspannung anlegbar ist, um elektrische Entladungen in dem Gasstrom zu erzeugen. Weiterhin können die Entladungsmittel eine Spannungsquelle, zum Beispiel einen Transformator, umfassen, um die Elektroden mit einer hochfrequenten Hochspannung zu beaufschlagen.

Die elektrische Entladung bewirkt in dem Gasstrom die Entstehung reaktiver Spezies. Bei den reaktiven Spezies kann es sich insbesondere um ein oder mehrere der folgenden Spezies handeln: Ozon, Stickoxide, Hydroxylradikale, Nitrite, Nitrate, vollständig oder teilweise ionisierte oder angeregte Atome oder Moleküle. Der Gasstrom kann durch die elektrische Entladung zumindest teilweise in den Plasmazustand versetzt werden.

Die oben genannte Aufgabe wird erfindungsgemäß weiterhin gelöst durch die Verwendung der zuvor beschriebenen Vorrichtung zur Desinfektion, insbesondere zur Sterilisation, von Gegenständen oder Feststoffen. Vorzugsweise wird die Vorrichtung verwendet zur Desinfektion, insbesondere zur Sterilisation, von Schutzausrüstungsteilen, insbesondere von Schutzmasken, wie zum Beispiel FFP2- oder FFP3-Masken, oder Schutzbekleidung (z. B. Tychem oder Mikrogard). Weiterhin kann die Vorrichtung zur Desinfektion, insbesondere zur Sterilisation, von anderen Gütern, insbesondere von Pulver, Saatgut oder Lebensmitteln, insbesondere von Gemüse, Obst, Salat, Nüssen wie zum Beispiel Haselnüssen, Mandeln, Hülsenfrüchten oder Gewürzen wie zum Beispiel Pfeffer, verwendet werden.

Im Folgenden werden verschiedene Ausführungsformen der Vorrichtung und der Verwendung beschrieben, wobei die einzelnen Ausführungsformen jeweils einzeln sowohl für die Vorrichtung als auch für die Verwendung gelten. Weiterhin können die einzelnen Ausführungsformen miteinander kombiniert werden.

Bei einer Ausführungsform sind die Entladungsmittel zur Erzeugung einer dielektrisch behinderten Entladung in dem Gasstrom eingerichtet. Durch eine dielektrisch behinderte Entladung können in dem Gasstrom sehr hohe Konzentrationen bestimmter reaktiver Spezies, insbesondere Ozon, erzeugt werden, wodurch eine starke desinfizierende, insbesondere sterilisierende Wirkung erreicht wird.

Die Entladungsmittel zur Erzeugung einer dielektrisch behinderten Entladung können insbesondere mindestens zwei Elektroden und ein dazwischen angeordnetes Dielektrikum umfassen, das eine direkte elektrische Entladung zwischen den zwei Elektroden behindert. Vorzugsweise ist eine der Elektroden geerdet. Weiterhin können die Entladungsmittel insbesondere eine Spannungsquelle aufweisen, um die Elektroden mit einer hochfrequenten Hochspannung zu beaufschlagen, beispielsweise mit einer Spannungsstärke im Bereich von 5 bis 15 kV und einer Spannungsfrequenz im Bereich von 7,5 bis 25 kHz, insbesondere 13 bis 14 kHz.

Gemäß Anspruch 1 sind die Entladungsmittel zur Erzeugung einer hochfrequenten Hochspannungsentladung, insbesondere zwischen mindestens zwei Elektroden, in dem Gasstrom eingerichtet. Erfindungsgemäß sind die Entladungsmittel zur Erzeugung einer bogenartigen Entladung in einem Gasstrom eingerichtet sein, wobei die bogenartige Entladung durch Anlegen einer hochfrequenten Hochspannung zwischen Elektroden erzeugt wird.

Insbesondere kann die Erzeugungseinheit eine im Betrieb mit dem Gasstrom durchströmte Plasmadüse zur Erzeugung eines atmosphärischen Plasmastrahls umfassen, wobei die Plasmadüse Entladungsmittel in Form von Elektroden aufweist, zwischen denen über eine dazu eingerichtete Hochspannungsquelle eine hochfrequente Hochspannung angelegt werden kann, so dass es zu hochfrequenten Hochspannungsentladungen, insbesondere zu einer hochfrequenten bogenartigen Entladung, kommt, durch die der durch die Plasmadüse geleitete Gasstrom mit reaktiven Spezies angereichert wird.

Auf diese Weise können in dem Gasstrom eine hohe Konzentration bestimmter reaktiver Spezies erzeugt werden, insbesondere Stickoxide und/oder vollständig oder teilweise ionisierte oder angeregte Atome oder Moleküle. Ozon wird gegenüber dielektrisch behinderten Entladungen nur zu einem geringen Anteil erzeugt, wodurch die Ozonbelastung reduziert wird. Demgegenüber werden durch eine hochfrequente bogenartige Entladung vermehrt Stickoxide erzeugt.

Unter einer hochfrequenten Hochspannung, insbesondere zur Erzeugung einer hochfrequenten bogenartigen Entladung, wird insbesondere eine Spannung von 1 - 100 kV, vorzugsweise 1 - 50 kV, weiter bevorzugt 10 - 50 kV, bei einer Frequenz von 1 - 300 kHz, insbesondere 1 - 100 kHz, vorzugsweise 10 - 100 kHz, weiter bevorzugt 10 - 50 kHz verstanden.

Bei einer weiteren Ausführungsform weist die Erzeugungseinheit eine erste Teilerzeugungseinheit und eine zweite Teilerzeugungseinheit auf, wobei die erste Teilerzeugungseinheit erste Entladungsmittel zur Erzeugung einer dielektrisch behinderten Entladung in einem ersten Teilgasstrom umfasst und wobei die zweite Teilerzeugungseinheit Entladungsmittel zur Erzeugung einer hochfrequenten Hochspannungsentladung in einem zweiten Teilgasstrom umfasst. Vorzugsweise ist die Erzeugungseinheit dazu eingerichtet, den ersten Teilgasstrom und den zweiten Teilgasstrom zusammenzuführen, insbesondere zu vermischen. Das Zusammenführen, insbesondere Vermischen, erfolgt vorzugsweise, bevor die Teilgasströme der Behandlungskammer zugeführt werden. Es wurde festgestellt, dass die erste Teilerzeugungseinheit mit sehr hoher Effizienz und Konzentration reaktive Spezies in dem ersten Teilgasstrom erzeugt. Weiterhin wurde festgestellt, dass das mit der ersten Teilerzeugungseinheit entstehende Ozon teilweise oder vollständig durch den zweiten Teilgasstrom aus der zweiten Teilerzeugungseinheit vernichtet werden kann. Auf diese Weise kann die Ozonbelastung reduziert werden, während der durch Mischen des ersten und zweiten Teilgasstroms entstandene gemeinsame Gasstrom weiterhin eine sterilisierende Wirkung hat.

Bei einer weiteren Ausführungsform ist die Erzeugungseinheit innerhalb der Behandlungskammer angeordnet. Auf diese Weise wird der Abstand zwischen den Entladungsmitteln und den im Betrieb in der Behandlungskammer angeordneten Gegenständen oder Feststoffen, vorzugsweise Schutzausrüstungsteilen, möglichst gering gehalten, wodurch eine hohe Desinfektionswirkung erreicht wird.

Bei einer weiteren Ausführungsform ist die Erzeugungseinheit in die Wand der Behandlungskammer integriert. Insbesondere kann ein Auslass der Erzeugungseinheit, aus der im Betrieb der Gasstrom mit den reaktiven Spezies ausstritt, in die Wand der Behandlungskammer integriert sein. Auf diese Weise kann der Abstand zwischen den Entladungsmitteln und den im Betrieb in der Behandlungskammer angeordneten Gegenständen oder Feststoffen, vorzugsweise Schutzausrüstungsteilen, gering gehalten, während die Erzeugungseinheit gleichzeitig vor der Atmosphäre in der Behandlungskammer weitgehend geschützt ist, wodurch deren Lebensdauer verlängert wird.

Bei einer weiteren Ausführungsform ist die Erzeugungseinheit außerhalb der Behandlungskammer angeordnet und derart an die Behandlungskammer angeschlossen ist, dass der Gasstrom im Betrieb in die Behandlungskammer gelangt. Auf diese Weise lässt sich die Erzeugungseinheit flexibler positionieren. Zudem kann auf diese Weise bereits durch die räumliche Anordnung sicher vermieden werden, dass ein Benutzer mit den Entladungsmittel der Erzeugungseinheit in Kontakt kommt, wodurch die Betriebssicherheit erhöht wird.

Bei einer Ausführungsform ist in der Behandlungskammer ein Ventilator angeordnet. Auf diese Weise kann im Betrieb der Gasstrom aus der Erzeugungseinheit besser in der Behandlungskammer verteilt werden, so dass in der Behandlungskammer angeordnete Gegenstände oder Feststoffe, vorzugsweise Schutzausrüstungsteile, gleichmäßiger desinfiziert werden.

Bei einer weiteren Ausführungsform sind in der Behandlungskammer ein oder mehrere Positioniervorrichtungen zur Positionierung von Gegenständen oder Feststoffen, vorzugsweise Schutzausrüstungsteilen, an vorgegebener Stelle in der Behandlungskammer angeordnet. Auf diese Weise kann sichergestellt werden, dass die Gegenstände oder Feststoffe, vorzugsweise Schutzausrüstungsteile, derart in der Behandlungskammer angeordnet werden, dass eine möglichst gleichmäßige und vollständige Desinfizierung der Gegenstände oder Feststoffe, vorzugsweise Schutzausrüstungsteile, erreicht wird. Als Positioniervorrichtung können beispielsweise ein oder mehrere Haken oder andere Haltevorrichtungen für Atemschutzmasken oder Schutzbekleidungsteile vorgesehen sein, die an vorbestimmten Positionen innerhalb der Behandlungskammer angeordnet sind.

Bei einer weiteren Ausführungsform ist in der Behandlungskammer ein rohrförmiges Element derart angeordnet, dass es im Betrieb von dem in die Behandlungskammer eingeleiteten Gasstrom durchströmt wird, wobei das rohrförmige Element dazu ausgebildet ist, mehrere Gegenstände, vorzugsweise Schutzausrüstungsteile, insbesondere mehrere Atemschutzmasken, derart aufzunehmen, dass die Gegenstände, vorzugsweise Schutzausrüstungsteile, insbesondere Atemschutzmasken, beim Durchströmen des rohrförmigen Elements mit dem Gasstrom durchströmt werden, insbesondere nacheinander. Es wurde festgestellt, dass sich Gegenstände, insbesondere Schutzausrüstungsteile wie Atemschutzmasken besser desinfizieren lassen, wenn diese mit einem reaktive Spezies enthaltenden Gasstrom zwangsdurchströmt werden. Durch das Vorsehen des rohrförmigen Elements wird der Gasstrom durch die darin angeordneten Gegenstände, vorzugsweise Schutzausrüstungsteile, insbesondere Atemschutzmasken, hindurchgeleitet, wodurch sich eine wesentlich bessere Desinfizierung erreichen lässt als durch ein diffuses Umströmen der Gegenstände, insbesondere Schutzausrüstungsteile, insbesondere Atemschutzmasken, mit reaktive Spezies aufweisendem Gas. Das rohrförmige Element kann insbesondere dazu eingerichtet sein, mehrere in Bezug auf die Durchströmungsrichtung hintereinander angeordnete Gegenstände, vorzugsweise Schutzausrüstungsteile, insbesondere Atemschutzmasken, aufzunehmen.

Bei einer weiteren Ausführungsform ist in der Behandlungskammer ein Lochblech derart angeordnet ist, dass es im Betrieb von dem in die Behandlungskammer eingeleiteten Gasstrom durchströmt wird, wobei das Lochblech ist insbesondere so angeordnet ist, dass mehrere Gegenstände, vorzugsweise mehrere Schutzausrüstungsteile, insbesondere mehrere Atemmasken, auf dem Lochblech positioniert werden können. Das Lochblech stellt auf diese Weise eine einfache Positioniervorrichtung für Gegenstände, vorzugsweise Schutzausrüstungsteile, insbesondere Atemmasken dar. Es wurde festgestellt, dass sich durch das Vorsehen eines Lochblechs zur Anordnung von Gegenständen, vorzugsweise Schutzausrüstungsteile, insbesondere Atemmasken, ein gezieltes Durchströmen der Gegenstände, vorzugsweise Schutzausrüstungsteile, mit dem Gasstrom erreichen lässt.

Vorzugsweise ist eine Absaugung, insbesondere umfassend einen Ventilator, vorgesehen, um den Gasstrom durch das Lochblech zu saugen. Auf diese Weise kann der Gasstrom mit höherem Durchsatz durch die auf dem Lochblech positionierten Gegenstände, vorzugsweise Schutzausrüstungsteile geleitet werden.

Bei einer weiteren Ausführungsform ist an der Behandlungskammer ein Auslass vorgesehen, um den Gasstrom aus der Behandlungskammer auszulassen. Auf diese Weise kann während oder am Ende eines Desinfektionsvorgangs Gas, insbesondere Gas mit reaktiven Spezies, aus der Behandlungskammer abgeführt werden, so dass die Belastung des Nutzers mit reaktiven Spezies, insbesondere Ozon, beim Öffnen der Schleuse reduziert wird. Zu diesem Zweck können an dem Auslass vorzugsweise Absaugungsmittel angeschlossen sein, die dazu eingerichtet sind, den Gasstrom aus der Behandlungskammer abzusaugen. Dadurch kann Gas mit reaktiven Spezies gezielt aus der Behandlungskammer entfernt werden. Vorzugsweise ist an der Behandlungskammer ein Frischlufteinlass vorgesehen, durch den insbesondere am Ende eines Desinfektionsvorgangs Frischluft beim Auslassen, insbesondere Absaugen, des Gasstroms aus der Behandlungskammer nachströmen kann.

Bei einer weiteren Ausführungsform ist an dem Auslass eine Neutralisationseinrichtung vorgesehen, die dazu eingerichtet ist, den Ozongehalt des Gasstroms, insbesondere des aus der Behandlungskammer ausgelassenen Gasstroms, zu reduzieren. Auf diese Weise kann die Ozonbelastung der Umgebung reduziert werden.

Die Neutralisationseinrichtung kann insbesondere eine Plasmadüse zur Erzeugung eines atmosphärischen Plasmastrahls umfassen. Es wurde festgestellt, dass sich der Ozongehalt im Gasstrom mit einem atmosphärischen Plasmastrahl erheblich reduzieren lässt. Die Plasmadüse ist vorzugsweise dazu eingerichtet ist, einen Plasmastrahl durch hochfrequente Hochspannungsentladungen in einem Arbeitsgasstrom zu erzeugen. Ein auf diese Weise erzeugter Plasmastrahl reduziert das Ozon sehr effektiv. Zur Reduzierung des Ozongehalts in dem aus der Behandlungskammer ausgelassenen Gasstrom kann der Gasstrom insbesondere als Arbeitsgasstrom durch die Plasmadüse geleitet werden. Alternativ dazu ist es auch denkbar, den aus der Behandlungskammer ausgelassenen Gasstrom mit dem aus der Plasmadüse austretenden Plasmastrahl zu beaufschlagen.

Bei einer weiteren Ausführungsform ist ein Umluftsystem vorgesehen, das dazu eingerichtet ist, den Gasstrom aus der Behandlungskammer abzuleiten und der Behandlungskammer über ein Umluftleitungssystem wieder zuzuleiten. Auf diese Weise können in dem Gasstrom wiederholt reaktive Spezies erzeugt werden, so dass insgesamt eine höhere Konzentration der reaktiven Spezies im Gasstrom erreicht wird. Zudem kann durch die mit dem Umluftsystem bewirkte Zirkulation des Gasstroms eine bessere Verteilung des Gasstroms mit den reaktiven Spezies in der Behandlungskammer erreicht werden. Das Umluftsystem umfasst insbesondere mindestens einen Umluft-Einlass an der Behandlungskammer, durch den der Gasstrom aus der Behandlungskammer in das Umluftleitungssystem gelangt, sowie mindestens einen Umluft-Auslass an der Behandlungskammer, durch den der Gasstrom aus dem Umluftleitungssystem wieder in die Behandlungskammer gelangt.

Bei einer weiteren Ausführungsform weist das Umluftsystem einen Ventilator auf, insbesondere einen Seitenkanalverdichter, der dazu eingerichtet ist, den Gasstrom aus der Behandlungskammer abzusaugen und durch das Umluftleitungssystem zu leiten. Auf diese Weise wird ein steuerbarer Gasstrom im Umluftsystem sichergestellt. Der Ventilator kann insbesondere in dem Umluftleitungssystem angeordnet sein.

Bei einer weiteren Ausführungsform ist die Erzeugungseinheit derart in das Umluftsystem integriert, dass der durch das Umluftsystem geleitete Gasstrom zumindest teilweise der Erzeugungseinheit zugeführt wird. Auf diese Weise wird der zirkulierte Gasstrom, der typischerweise noch einige reaktive Spezies enthält, durch die Erzeugungseinheit weiter mit reaktiven Spezies angereichert. Die Erzeugungseinheit kann zwischen einem ersten und einem zweiten Abschnitt des Umluftleitungssystems angeordnet sein, wobei der erste Abschnitt den Gasstrom von der Behandlungskammer zur Erzeugungseinheit und der zweite Abschnitt den Gasstrom von der Erzeugungseinheit zurück zur Behandlungskammer leitet. Alternativ dazu kann die Erzeugungseinheit auch am Ende des Umluftleitungssystems angeordnet sein, so dass der Gasstrom aus der Erzeugungseinheit in die Behandlungskammer gelangt. Es ist weiterhin denkbar, dass die Erzeugungseinheit in einer von einer Hauptleitung des Umluftleitungssystems abzweigenden Bypass-Leitung angeordnet ist, wobei die Bypass-Leitung stromabwärtsseitig der Erzeugungseinheit wieder in die Hauptleitung oder direkt in die Behandlungskammer mündet.

Bei einer weiteren Ausführungsform ist die Erzeugungseinheit separat vom Umluftsystem ausgebildet. Vorzugsweise weist die Erzeugungseinheit zu diesem Zweck eine vom Umluftsystem separate Zuleitung für die Versorgung der Erzeugungseinheit mit einem separaten Gasstrom auf, zum Beispiel mit einer separaten Frischluftversorgung. Durch die Trennung des Umluftsystems und der Erzeugungseinheit kann die Lebensdauer der Erzeugungseinheit verbessert werden, da sie weniger Kontakt zu den reaktiven Spezies im zirkulierten Gasstrom hat.

Erfindungsgemäß sind Befeuchtungsmittel vorgesehen, die dazu eingerichtet sind, die relative Feuchtigkeit des Gasstroms und/oder die relative Feuchtigkeit in der Behandlungskammer zu erhöhen, vorzugsweise auf eine relative Feuchtigkeit im Bereich von 90 % rF - 100 % rF, vorzugsweise 95 % rF - 98 % rF. Es wurde festgestellt, dass sich durch die Erhöhung der relativen Feuchtigkeit, insbesondere in den genannten Bereichen, in Kombination mit den reaktiven Spezies im Gasstrom eine bessere Desinfektionswirkung erzielen lässt. Die relative Feuchtigkeit des Gasstroms wird insbesondere erhöht, bevor er in den für die Gegenstände oder Feststoffe, vorzugsweise Schutzausrüstungsteile, vorgesehenen Bereich der Behandlungskammer gelangt.

Bei einer weiteren Ausführungsform umfassen die Befeuchtungsmittel eine vorzugsweise beheizbare Wasserwanne. Auf diese Weise kann die vorteilhafte Erhöhung der relativen Feuchtigkeit auf einfache Weise bewirkt werden. Die Wasserwanne kann insbesondere in der Behandlungskammer angeordnet sein. Es ist jedoch auch denkbar, die Wasserwanne außerhalb der Behandlungskammer anzuordnen, insbesondere wenn die Erzeugungseinheit außerhalb der Behandlungskammer angeordnet ist.

Es wurde jedoch festgestellt, dass sich eine Erhöhung der relativen Feuchtigkeit im Gasstrom bereits mit einer unbeheizten Wasserwanne, insbesondere mit Wasser bei Raumtemperatur, erreichen lässt. Um zum Beispiel Kosten zu sparen, kann daher auf Heizmittel verzichtet werden.

Um eine höhere Verdunstungsleistung zu erreichen, sind jedoch vorzugsweise Heizmittel zum Beheizen der Wasserwanne vorgesehen, die besonders bevorzugt dazu eingerichtet sind, in der Wasserwanne enthaltenes Wasser auf eine vorgegebene Solltemperatur einzuregeln, insbesondere im Bereich von 50 - 100 °C, vorzugsweise 50 - 80 °C. Auf diese Weise kann mehr Wasserdampf erzeugt und die relative Feuchtigkeit des Gasstrom effektiver zum Beispiel auch bei höherem Gasstromdurchsatz erhöht werden.

Bei einer weiteren Ausführungsform ist die Wasserwanne derart angeordnet, dass der Gasstrom von der Erzeugungseinheit im Betrieb auf die Wasserwanne gerichtet wird. Zu diesem Zweck kann beispielsweise eine Leitung oder eine Düsenöffnung vorgesehen sein, durch die der Gasstrom im Betrieb auf die Wasserwanne gerichtet wird. Es wurde festgestellt, dass sich die relative Feuchtigkeit des Gasstroms sehr einfach und effektiv dadurch erhöhen lässt, dass der Gasstrom über die Wasseroberfläche des in der Wasserwanne befindlichen flüssigen Wassers geblasen wird. Auf diese Weise reichert sich der über der Wasseroberfläche befindliche Wasserdampf in dem Gasstrom an.

Bei einer weiteren Ausführungsform ist in der Wasserwanne ein Verdunstungskörper angeordnet. Auf diese Weise kann der Gasstrom effektiver befeuchtet werden. Zum einen kann mit einem solchen Verdunstungskörper eine höhere Verdunstungsleistung erreicht werden. Zum anderen begünstigt ein solcher Verdunstungskörper, dass kleine Wassertröpfchen mit dem Gasstrom mitgerissen werden, so dass sich der Wassergehalt des Gasstroms auf diese Weise erhöht. Der Verdunstungskörper besteht vorzugsweise aus einem porösen, insbesondere schwammartigen Material. Derartige Materialien weisen in Bezug auf ihr Volumen eine sehr große Oberfläche auf, wodurch die Befeuchtung des Gasstroms verbessert wird. Der Verdunstungskörper ist vorzugsweise derart angeordnet, dass er im Betrieb von dem Gasström umströmt und/oder durchströmt wird.

Bei einer weiteren Ausführungsform ist in der Wasserwanne ein Vernebler, insbesondere ein Ultraschallvernebler angeordnet. Auf diese Weise kann die Verdunstungsleistung ebenfalls verbessert werden.

Bei einer weiteren Ausführungsform ist die Wasserwanne mit plasmaaktiviertem Wasser befüllt. Auf diese Weise kann die Desinfektionswirkung weiter verbessert werden.

Unter plasmaaktiviertem Wasser wird Wasser verstanden, das durch die Einwirkung von einem aus einer atmosphärischen Plasmaquelle austretenden Arbeitsgas aktiviert worden ist. Insbesondere kann das Wasser unmittelbar mit atmosphärischem Plasma, wie zum Beispiel einem atmosphärischen Plasmastrahl, beaufschlagt werden, das heißt mit einem aus der Plasmaquelle austretenden Arbeitsgas, das sich zumindest teilweise noch im Plasmazustand befindet. Weiterhin kann das Wasser auch mit dem aus der Plasmaquelle austretenden Arbeitsgas beaufschlagt werden, nachdem das Arbeitsgas bereits wieder rekombiniert ist, das heißt sich nicht mehr im Plasmazustand befindet. Es wurde festgestellt, dass auch in einem solchen rekombinierten Arbeitsgas noch ausreichend reaktive Spezies, beispielsweise Ozon oder Stickoxide, enthalten sind, die im Wasser relativ langlebige reaktive Spezies wie zum Beispiel Wasserstoffperoxid, Salpetersäure oder salpetrige Säure bilden.

Entsprechend kann das plasmaaktivierte Wasser durch Einwirkung eines aus einer atmosphärischen Plasmaquelle austretenden Arbeitsgases auf (insbesondere flüssiges) Wasser hergestellt werden bzw. hergestellt sein.

Geeignetes plasmaaktiviertes Wasser und eine Vorrichtung zu dessen Herstellung wird zum Beispiel in der EP 3 470 364 A1 beschrieben.

Bei der Verwendung von plasmaaktiviertem Wasser ist insbesondere das Vorsehen eines Verdunstungskörpers oder eines Verneblers in der Wasserwanne vorteilhaft, denn diese begünstigen, dass Wassertröpfchen mit dem Gasstrom mitgerissen werden. Gegenüber einem reinen Verdampfen des Wassers hat das Befeuchten des Gasstroms mit Wassertröpfchen den Vorteil, dass die reaktiven Spezies aus dem plasmaaktiviertem Wasser in größerem Umfang in den Gasstrom gelangen.

Weitere Merkmale und Vorteile der Vorrichtung und der Verwendung ergeben sich aus der folgenden Beschreibung von Ausführungsbeispielen, wobei auf die beigefügte Zeichnung Bezug genommen wird.

In der Zeichnung zeigen
- Fig. 1: eine Plasmadüse zur Erzeugung eines atmosphärischen Plasmastrahls,
- Fig. 2: eine Düse zur Erzeugung reaktiver Spezies in einem Gasstrom mittels dielektrisch behinderter Entladung,
- Fig. 3: ein Ausführungsbeispiel der Vorrichtung zur Desinfektion von Gegenständen oder Feststoffen, vorzugsweise Schutzausrüstungsteilen, sowie für dessen Verwendung,
- Fig. 4: die Erzeugungseinheit der Vorrichtung aus Fig. 3,
- Fig. 5: eine alternative Erzeugungseinheit für die Vorrichtung aus Fig. 3,
- Fig. 6: eine weitere alternative Erzeugungseinheit für die Vorrichtung aus Fig. 3,
- Fig. 7: eine weitere alternative Erzeugungseinheit für die Vorrichtung aus Fig. 3,
- Fig. 8: ein weiteres Ausführungsbeispiel der Vorrichtung zur Desinfektion von Gegenständen oder Feststoffen, vorzugsweise Schutzausrüstungsteilen, sowie für dessen Verwendung,
- Fig. 9: die Neutralisationseinrichtung der Vorrichtung aus Fig. 8,
- Fig. 10 .: eine alternative Neutralisationseinrichtung für die Vorrichtung aus Fig. 8,
- Fig. 11: ein weiteres Ausführungsbeispiel der Vorrichtung zur Desinfektion von Gegenständen oder Feststoffen, vorzugsweise Schutzausrüstungsteilen, sowie für dessen Verwendung,
- Fig. 12: ein weiteres Ausführungsbeispiel der Vorrichtung zur Desinfektion von Gegenständen oder Feststoffen, vorzugsweise Schutzausrüstungsteilen, sowie für dessen Verwendung,
- Fig. 13: ein weiteres Ausführungsbeispiel der Vorrichtung zur Desinfektion von Gegenständen oder Feststoffen, vorzugsweise Schutzausrüstungsteilen, sowie für dessen Verwendung,
- Fig. 14: ein weiteres Ausführungsbeispiel der Vorrichtung zur Desinfektion von Gegenständen oder Feststoffen, vorzugsweise Schutzausrüstungsteilen, sowie für dessen Verwendung,
- Fig. 15: ein weiteres Ausführungsbeispiel der Vorrichtung zur Desinfektion von Gegenständen oder Feststoffen, vorzugsweise Schutzausrüstungsteilen, sowie für dessen Verwendung,
- Fig. 16a-b: ein weiteres Ausführungsbeispiel der Vorrichtung zur Desinfektion von Gegenständen oder Feststoffen, vorzugsweise Schutzausrüstungsteilen, sowie für dessen Verwendung,
- Fig. 17: eine weitere alternative Erzeugungseinheit für die Vorrichtung aus Fig. 3 und
- Fig. 18: eine weitere alternative Erzeugungseinheit für die Vorrichtung aus Fig. 3.

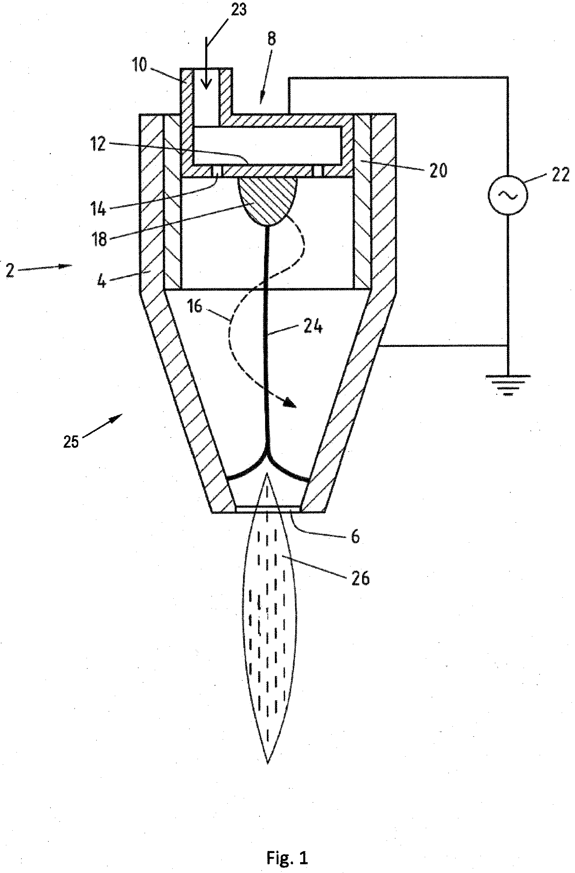

Fig. 1 zeigt eine Plasmadüse 2 zur Erzeugung eines atmosphärischen Plasmastrahls 26.

Die Plasmadüse 2 weist ein Düsenrohr 4 aus Metall auf, das sich konisch zu einer Düsenöffnung 6 verjüngt. An dem der Düsenöffnung 6 entgegen gesetzten Ende weist das Düsenrohr 4 eine Dralleinrichtung 8 mit einem Einlass 10 für einen Gasstrom, insbesondere eines Arbeitsgases, beispielsweise Stickstoff, auf.

Eine Zwischenwand 12 der Dralleinrichtung 8 weist einen Kranz von schräg in Umfangsrichtung angestellten Bohrungen 14 auf, durch die der Gasstrom verdrallt wird. Der stromabwärtige, konisch verjüngte Teil des Düsenrohres wird deshalb von dem Gasstrom in der Form eines Wirbels 16 durchströmt, dessen Kern auf der Längsachse des Düsenrohres verläuft. An der Unterseite der Zwischenwand 12 ist mittig eine Elektrode 18 angeordnet, die koaxial in Richtung des verjüngten Abschnittes in das Düsenrohr hineinragt. Die Elektrode 18 ist elektrisch mit der Zwischenwand 12 und den übrigen Teilen der Dralleinrichtung 8 verbunden. Die Dralleinrichtung 8 ist durch ein Keramikrohr 20 elektrisch gegen das Düsenrohr 4 isoliert. Über die Dralleinrichtung 8 wird an die Elektrode 18 eine hochfrequente Hochspannung angelegt, die von einem Transformator 22 erzeugt wird. Der Einlass 10 wird über eine nicht gezeigte Leitung mit einem Gasstrom 23 versorgt. Das Düsenrohr 4 ist geerdet. Durch die angelegte Spannung wird eine Hochfrequenzentladung in der Form eines Lichtbogens 24 zwischen der Elektrode 18 und dem Düsenrohr 4 erzeugt. Die mit dem Transformator verbundene Elektrode 18 und das geerdete Düsenrohr 4 stellen damit Entladungsmittel 25 dar, die zur Erzeugung einer hochfrequenten Hochspannungsentladung in Form des Lichtbogens 24 in einem Gasstrom 23 eingerichtet sind.

Die Begriffe "Lichtbogen", "Bogenentladung" bzw. "bogenartige Entladung" werden vorliegend als phänomenologische Beschreibung der Entladung verwendet, da die Entladung in Form eines Lichtbogens auftritt. Der Begriff "Lichtbogen" wird anderweitig auch als Entladungsform bei Gleichspannungsentladungen mit im Wesentlichen konstanten Spannungswerten verwendet. Vorliegend handelt es sich jedoch um eine Hochfrequenzentladung in Form eines Lichtbogens, also um eine hochfrequente, bogenartige Entladung.

Aufgrund der drallförmigen Strömung des Arbeitsgases wird dieser Lichtbogen jedoch im Wirbelkern auf der Achse des Düsenrohres 4 kanalisiert, so dass er sich erst im Bereich der Düsenöffnung 6 zur Wand des Düsenrohres 4 verzweigt. Das Arbeitsgas, das im Bereich des Wirbelkerns und damit in unmittelbarer Nähe des Lichtbogens 24 mit hoher Strömungsgeschwindigkeit rotiert, kommt mit dem Lichtbogen in innige Berührung und wird dadurch zum Teil in den Plasmazustand überführt, so dass ein atmosphärischer Plasmastrahl 26 durch die Düsenöffnung 6 aus der Plasmadüse 2 austritt.

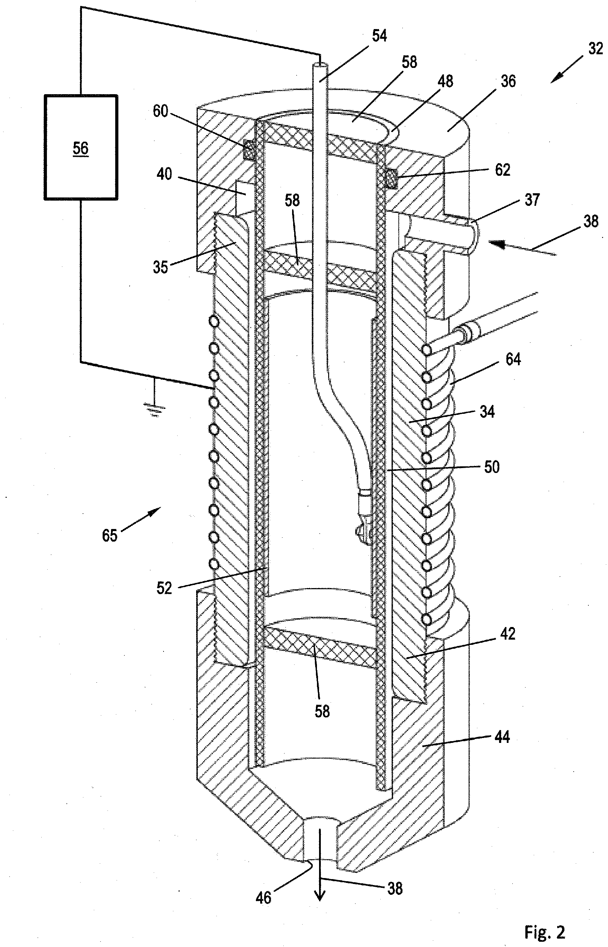

Fig. 2 zeigt eine Düse zur Erzeugung reaktiver Spezies in einem Gasstrom mittels dielektrisch behinderter Entladung.

Die Düse 32 weist ein Düsenrohr 34 aus Metall auf, an dessen stromaufwärtsseitigem Ende 35 ein Verteilerkopf 36 mit einem Einlass 37 für einen Gasstrom 38, insbesondere für einen Arbeitsgasstrom, und mit einem ringförmigen Verteilerkanal 40 angeordnet ist. Am gegenüberliegenden stromabwärtsseitigen Ende 42 des Düsenrohrs 34 ist eine Auslassdüse 44 mit einer Düsenöffnung 46 angeordnet, aus der im Betrieb der mit reaktiven Spezies angereicherte Gasstrom 38 austritt.

Vom Verteilerkopf 36 erstreckt sich ein Keramikrohr 48 derart durch das Düsenrohr 34 bis in die Auslassdüse 44, dass sich von dem Verteilerkanal 40 ein ringförmiger Entladungskanal 50 zwischen dem Düsenrohr 34 und dem Keramikrohr 48 bis zur Auslassdüse 44 erstreckt. Anstelle eines Keramikrohrs käme zum Beispiel auch ein Rohr aus Quarzglas in Frage.

Auf der Innenseite des Keramikrohrs 48 ist eine rohrförmige Hochspannungselektrode 52 aus Metall angeordnet, die über ein Hochspannungskabel 54 mit einem Transformator 56 verbunden ist, mit dem zwischen der Hochspannungselektrode 52 und dem als Gegenelektrode wirkenden, geerdeten Düsenrohr 34 eine hochfrequente Hochspannung angelegt werden kann. Anstelle einer rohrförmigen Hochspannungselektrode 52 käme zum Beispiel auch eine anders geformte Hochspannungselektrode in Betracht, beispielsweise in Form eines gerundeten Blechs.

Im Keramikrohr 48 sind isolierende Stopfen 58 angeordnet, die die Hochspannungselektrode 52 einschließen und weiterhin verhindern, dass Arbeitsgas in den Bereich der Hochspannungselektrode 52 strömt oder durch das Keramikrohr 48 aus der Düse 32 herausströmt. Weiterhin ist ein Dichtungsring 60 in eine ringförmige Nut 62 am Verteilerkopf 36 eingesetzt, die den Verteilerkopf 36 zum Keramikrohr 48 abdichtet.

Um das Düsenrohr 34 kann eine Kühlmittelleitung 64 vorgesehen sein, durch die im Betrieb ein Kühlmittel zur Kühlung des Düsenrohrs 34 geleitet werden kann. Die Kühlmittelleitung 64 kann zum Beispiel wie dargestellt spiralförmig um das Düsenrohr 34 verlaufen.

Im Betrieb wird ein Gasstrom 38 durch den Einlass 37 in den Verteilerkopf 36 eingeleitet, so dass der Gasstrom 38 durch den ringförmigen Entladungskanal 50 strömt.

Mit dem Transformator 56 wird zwischen der Hochspannungselektrode 52 und dem Düsenrohr 34 eine hochfrequente Hochspannung angelegt, so dass es im Entladungskanal 50 im Bereich der Hochspannungselektrode 52 zu dielektrisch behinderten Entladungen kommt, durch die in dem dort strömenden Gasstrom 38 reaktive Spezies, insbesondere Ozon, erzeugt werden. Die mit dem Generator 56 verbundene Hochspannungselektrode 52 und das geerdete Düsenrohr 34 stellen damit Entladungsmittel 65 dar, die zur Erzeugung einer dielektrisch behinderten Entladung in einem Gasstrom 38 eingerichtet sind.

Der mit den reaktiven Spezies angereicherte Gasstrom 38 tritt aus der Düsenöffnung 46 aus.

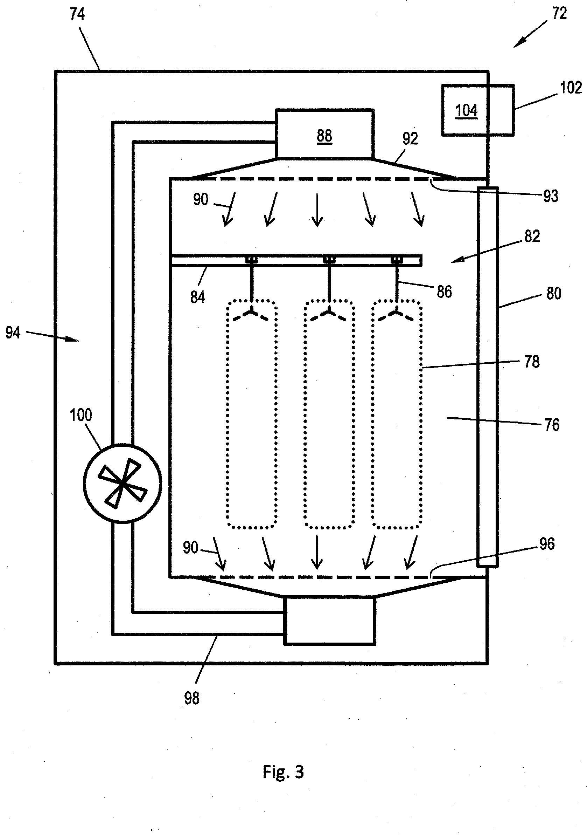

Fig. 3 zeigt ein Ausführungsbeispiel der Vorrichtung zur Desinfektion von Gegenständen oder Feststoffen, vorzugsweise Schutzausrüstungsteilen, sowie für dessen Verwendung.

Die Vorrichtung 72 weist ein Gehäuse 74 auf, in dem eine Behandlungskammer 76 zur Aufnahme von Schutzausrüstungsteilen wie zum Beispiel Schutzmasken oder Schutzbekleidung 78 angeordnet ist. An einer Seite der Behandlungskammer 76 ist eine verschließbare Schleuse 80 in Form einer am Gehäuse 74 befestigten Tür vorgesehen, durch die ein Nutzer zu desinfizierende Schutzausrüstungsteile in der Behandlungskammer 76 anordnen bzw. nach Ende des Desinfektionsvorgangs die desinfizierten Schutzausrüstungsteile aus der Behandlungskammer 76 entnehmen kann.

In der Behandlungskammer 76 sind Positioniervorrichtungen 82 vorgesehen, mit denen Schutzausrüstungsteile an vorgegebener Stelle in der Behandlungskammer 76 angeordnet werden können. Im vorliegenden Ausführungsbeispiel sind die Positioniervorrichtungen 82 in Form einer Halterung 84 mit mehreren Kleiderbügeln 86 zum Aufhängen von Schutzbekleidung 78 ausgebildet. Auf diese Weise kann eine Faltenbildung der Schutzbekleidung 78 verhindert werden und die Schutzbekleidungsteile 78 weisen einen vorgegebenen Abstand zueinander auf, so dass eine gleichmäßigere und zuverlässigere Desinfektion ermöglicht wird.

Die Vorrichtung 72 umfasst weiterhin eine Erzeugungseinheit 88 zur Erzeugung reaktiver Spezies in einem Gasstrom 90. Die Erzeugungseinheit 88 ist oberhalb der Behandlungskammer 76 angeordnet und mit dieser über einen Verteiler 92 mit mehreren Öffnungen 93 verbunden, durch die der von der Erzeugungseinheit 88 mit reaktiven Spezies angereicherte Gasstrom 90 im Betrieb in die Behandlungskammer 76 strömt und eine Desinfektion der in der Behandlungskammer 76 angeordneten Schutzausrüstungsteile bewirkt.

Die Erzeugungseinheit 88 ist in ein Umluftsystem 94 integriert, das am Boden der Behandlungskammer 76 Umluft-Einlässe 96 aufweist, durch die der Gasstrom 90 nach Durchlaufen der Behandlungskammer 76 in ein Umluftleitungssystem 98 gelangt. In dem Umluftleitungssystem 98 ist ein Ventilator 100, insbesondere ein Seitenkanalverdichter, angeordnet, der den Gasstrom 90 aus der Behandlungskammer 76 durch die Umluft-Einlässe 96 absaugt und wieder der Erzeugungseinheit 88 zuführt.

Da der Gasstrom 90 auch nach Durchlaufen der Behandlungskammer 76 typischerweise noch reaktive Spezies enthält, kann durch das Umluftsystem 94 eine sukzessive Erhöhung der reaktiven Spezies im Gasstrom 90 erreicht werden, wodurch die Desinfektionswirkung verbessert wird.

Die Vorrichtung 72 kann weiterhin eine mit einer Steuereinrichtung 104 verbundene Bedieneinheit 102 aufweisen, über die ein Nutzer die Vorrichtung 72 bedienen kann. Die Steuereinrichtung 104 ist dazu eingerichtet, die verschiedenen Komponenten der Vorrichtung 72, insbesondere die Erzeugungseinheit 88 und den Ventilator 100 abhängig von der über die Bedieneinheit 102 empfangener Nutzereingaben zu steuern.

Um die Vorrichtung 72 zur Desinfektion von Schutzausrüstungsteilen zu verwenden, öffnet der Nutzer zunächst die Schleuse 80 und positioniert die zu desinfizierenden Schutzausrüstungsteile, zum Beispiel die in Fig. 3 dargestellten Schutzbekleidungsteile 78, mittels der dafür vorgesehenen Positioniervorrichtungen 82. Anschließend schließt der Nutzer die Schleuse 80 und aktiviert die Vorrichtung 72 über die Bedieneinheit 102. Die Steuereinrichtung 104 steuert daraufhin die Erzeugungseinheit 88 und den Ventilator 100 derart an, dass die Erzeugungseinheit 88 reaktive Spezies in dem Gasstrom 90 erzeugt, der durch den Ventilator durch die Behandlungskammer 76 und das Umluftsystem 94 geführt wird. Nach Ablauf einer vorgegebenen Zeit, deaktiviert die Steuereinrichtung die Erzeugungseinheit 88 und den Ventilator 100. Der Nutzer kann der Behandlungskammer 76 dann nach Öffnen der Schleuse 80 die desinfizierten Schutzausrüstungsteile entnehmen.

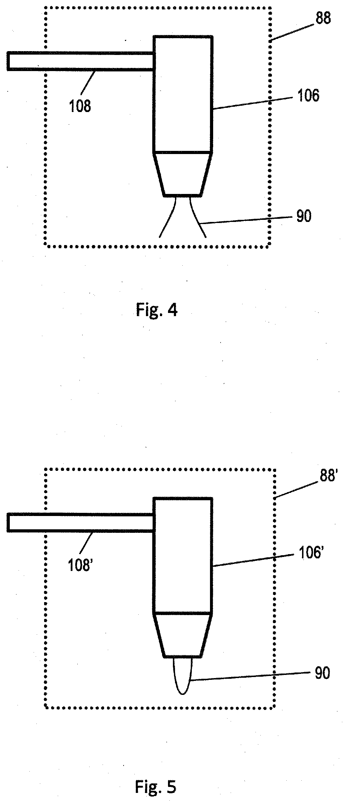

Fig. 4 zeigt die Erzeugungseinheit 88 der Vorrichtung 72 aus Fig. 3. Die Erzeugungseinheit 88 umfasst eine Düse 106 zur Erzeugung reaktiver Spezies in dem durch die Düse 106 geleiteten Gasstrom 90, wobei die Düse 106 Entladungsmittel aufweist, die zur Erzeugung einer dielektrisch behinderten Entladung in dem Gasstrom 90 eingerichtet sind, der der Düse 106 über eine Zuleitung 108 zugeführt wird. Die Düse 106 kann insbesondere wie die in Fig. 2 dargestellte Düse 32 ausgebildet sein. Die Zuleitung 108 ist bei der Vorrichtung 72 aus Fig. 3 mit dem Umluftleitungssystem 98 verbunden, so dass der aus der Behandlungskammer 76 abgesaugte Gasstrom 90 der Düse 106 wieder zugeführt wird. Die Verwendung von Entladungsmitteln zur Erzeugung einer dielektrisch behinderten Entladung bewirkt einen Gasstrom 90 mit hoher Ozonkonzentration und damit guter Desinfektionswirkung.

Fig. 5 zeigt eine alternative Erzeugungseinheit 88', die anstelle der Erzeugungseinheit 88 für die Vorrichtung aus Fig. 3 verwendet werden kann. Die Erzeugungseinheit 88' umfasst eine Düse 106' zur Erzeugung reaktiver Spezies im Gasstrom 90, wobei die Düse 106' Entladungsmittel aufweist, die zur Erzeugung einer hochfrequenten Hochspannungsentladung in dem Gasstrom 90 eingerichtet sind, der der Düse 106' über die Zuleitung 108' zugeführt wird. Die Düse 106' kann insbesondere wie die in Fig. 1 dargestellte Plasmadüse 2 ausgebildet sein, so dass der Gasstrom 90 in Form eines Plasmastrahls aus der Düse 106' austritt. Bei Verwendung der Erzeugungseinheit 88' für die Vorrichtung 72 aus Fig. 3 ist die Zuleitung 108' mit dem Umluftleitungssystem 98 verbunden, so dass der aus der Behandlungskammer 76 abgesaugte Gasstrom 90 der Düse 106' wieder zugeführt wird. Die Verwendung von Entladungsmitteln zur Erzeugung einer hochfrequenten Hochspannungsentladung bewirkt einen Gasstrom 90 mit reaktiven Spezies, jedoch mit geringer Ozonkonzentration, wodurch eine Ozonbelastung der Nutzer oder der Umwelt reduziert werden kann.

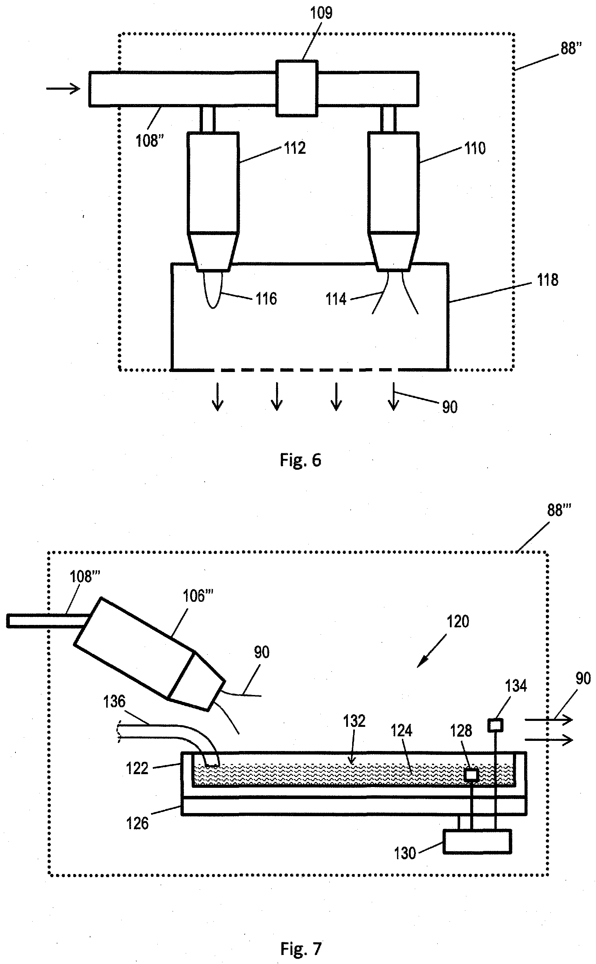

Fig. 6 zeigt eine weitere alternative Erzeugungseinheit 88", die anstelle der Erzeugungseinheit 88 für die Vorrichtung aus Fig. 3 verwendet werden kann. Die Erzeugungseinheit 88" umfasst eine erste Teilerzeugungseinheit 110 und eine zweite Teilerzeugungseinheit 112, die im vorliegenden Beispiel über eine gemeinsame Zuleitung 108" mit einem jeweiligen Teilgasstrom versorgt werden. Wenn die Teilerzeugungseinheiten 110, 112 zum Betrieb unterschiedliche Gasstromdurchsätze erfordern, kann wie in Fig. 6 dargestellt die Teilerzeugungseinheit 110 mit dem höheren Gasdurchsatz stromaufwärts der Teilerzeugungseinheit 112 mit dem geringeren Gasdurchsatz von der Zuleitung 108" abzweigen und vor der Teilerzeugungseinheit 112 ein Drosselventil 109 vorgesehen werden.

Bei Verwendung der Erzeugungseinheit 88" für die Vorrichtung 72 aus Fig. 3 ist die Zuleitung 108' mit dem Umluftleitungssystem 98 verbunden, so dass ein jeweiliger Teil des aus der Behandlungskammer 76 abgesaugten Gasstroms 90 der ersten und zweiten Teilerzeugungseinheit 110, 112 als jeweiliger Teilgasstrom zugeführt wird.

Die erste Teilerzeugungseinheit 110 umfasst erste Entladungsmittel zur Erzeugung einer dielektrisch behinderten Entladung in dem ersten Teilgasstrom und kann insbesondere wie die Düse 32 aus Fig. 2 ausgebildet sein. Die zweite Teilerzeugungseinheit 112 umfasst zweite Entladungsmittel zur Erzeugung einer hochfrequenten Hochspannungsentladung in dem zweiten Teilgasstrom und kann insbesondere wie die Plasmadüse 2 aus Fig. 1 ausgebildet sein. Im Betrieb tritt aus der ersten Teilerzeugungseinheit 110 der erste mit reaktiven Spezies angereicherte Teilgasstrom 114 und aus der zweiten Teilerzeugungseinheit 110 der zweite mit reaktiven Spezies angereicherte Teilgasstrom 116 aus. Der erste und der zweite Teilgasstrom 114, 116 werden in einer Mischkammer 118 zusammengeführt und vermischt, so dass der daraus erhaltende gemeinsamer Gasstrom 90 mit reaktiven Spezies aus der Mischkammer 118 austritt.

Es wurde festgestellt, dass der hohe Ozongehalt des mit der ersten Teilerzeugungseinheit 110 erzeugten ersten Teilgasstroms 114 durch das Vermischen mit dem zweiten Teilgasstrom 116 der zweiten Teilerzeugungseinheit 112 deutlich reduziert werden kann, wodurch ein weiterhin mit reaktiven Spezies angereicherter, gemeinsamer Gasstrom 90 mit reduzierter Ozonbelastung für Nutzer um Umwelt erreicht wird.

Fig. 7 zeigt eine weitere alternative Erzeugungseinheit 88‴, die anstelle der Erzeugungseinheit 88 für die Vorrichtung aus Fig. 3 verwendet werden kann. Die Erzeugungseinheit 88‴ umfasst eine Düse 106"', die zum Beispiel wie die Düse 32 aus Fig. 2 oder die Plasmadüse 2 aus Fig. 1 ausgebildet sein kann. Bei Verwendung der Erzeugungseinheit 88‴ für die Vorrichtung 72 aus Fig. 3 ist die Zuleitung 108‴ mit dem Umluftleitungssystem 98 verbunden, so dass der aus der Behandlungskammer 76 abgesaugte Gasstrom 90 der Düse 106‴ als Arbeitsgas zugeführt wird. Anstelle der Düse 106"' kann bei der Erzeugungseinheit 88‴ auch eine Kombination zweier Düsen wie in Fig. 6 mit einer Mischkammer 118 verwendet werden.

Weiterhin sind Befeuchtungsmittel 120 zur Befeuchtung des aus der Düse 106‴ austretenden Gasstroms 90 vorgesehen. Die Befeuchtungsmittel 120 umfassen eine Wasserwanne 122, die im Betrieb mit Wasser 124 befüllt ist. An der Wasserwanne 122 sind Heizmittel 126, beispielsweise in Form einer Heizplatte, sowie ein Temperatursensor 128 zum Messen der Wassertemperatur vorgesehen. Mittels vorgesehener Steuermittel 130 kann das Wasser 124 in der Wasserwanne 122 auf diese Weise auf eine vorgegebene Temperatur eingeregelt werden.

Die Wasserwanne 122 ist so angeordnet, dass der aus der Düse 106"' austretende Gasstrom 90 auf die Wasserwanne 122 gerichtet ist und dadurch über die Wasseroberfläche 132 des Wassers 124 in der Wasserwanne 122 geblasen wird. Dadurch erhöht sich die relative Feuchtigkeit des Gasstroms 90, wodurch in der Behandlungskammer 76 eine bessere Desinfektionswirkung erreicht wird.

Es kann weiterhin ein Feuchtigkeitssensor 134 vorgesehen sein, der die relative Feuchtigkeit des über die Wasseroberfläche 132 geblasenen Gasstroms 90 erfasst. Auf diese Weise lässt sich die Temperatur des Wassers zum Beispiel so regeln, dass eine vorgegebene relative Feuchtigkeit erreicht wird, insbesondere im Bereich von 90 % rF - 100 % rF, vorzugsweise 95 % rF - 99 % rF. Mit einer relativen Feuchtigkeit knapp unterhalb von 100 % rF kann eine ggf. unerwünschte Kondensation reduziert oder verhindert werden.

Um die durch Verdunstung aus der Wasserwanne 122 verloren gegangene Wassermenge wieder aufzufüllen, kann eine Wasserzuleitung 136 vorgesehen sein.

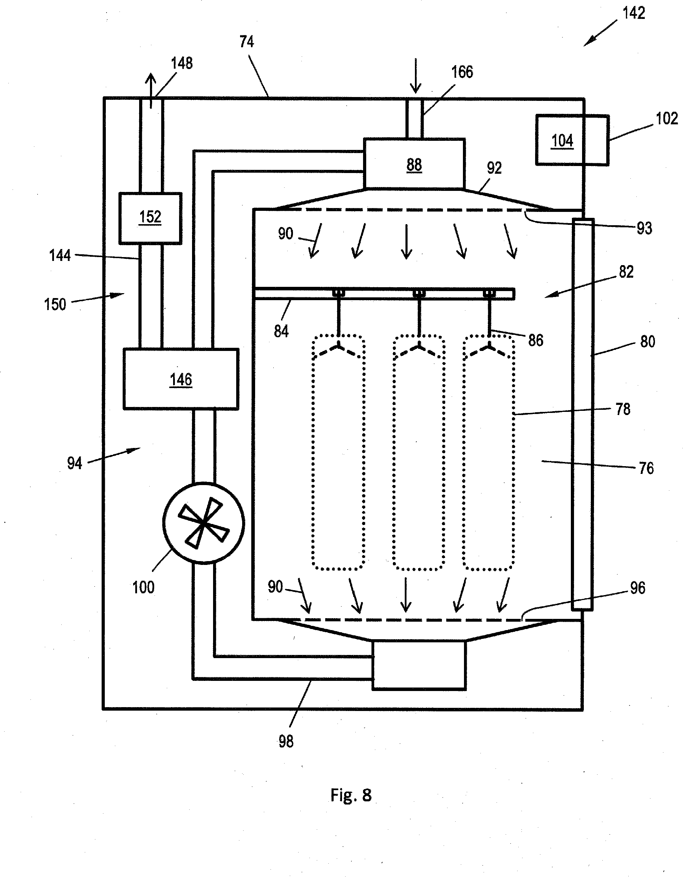

Fig. 8 zeigt ein weiteres Ausführungsbeispiel der Vorrichtung zur Desinfektion von Gegenständen oder Feststoffen, vorzugsweise Schutzausrüstungsteilen, sowie für dessen Verwendung. Die Vorrichtung 142 in Fig. 8 weist einen ähnlichen Aufbau auf wie die Vorrichtung 72 aus Fig. 3. Einander entsprechende Komponenten sind daher mit denselben Bezugszeichen versehen und es wird diesbezüglich auf die entsprechende Beschreibung zu Fig. 3 verwiesen.

Die Vorrichtung 142 unterscheidet sich dadurch von der Vorrichtung 72, dass von dem Umluftleitungssystems 98 eine Auslassleitung 144 abzweigt, durch die der im Umluftleitungssystem 98 geführte Gasstrom 90 durch Umschalten einer Steuerklappe 146 zu einer Auslassöffnung 148 geführt wird. Bei entsprechenden Stellung der Steuerklappe stellen die bodenseitigen Öffnungen mit dem daran anschließenden Teil des Umluftleitungssystems und der Auslassleitung 144 einen an der Behandlungskammer 76 vorgesehenen Auslass 150 dar, durch den der Gasstrom 90 aus der Behandlungskammer 76 ausgelassen werden kann. An dem Auslass 150 ist eine Neutralisationseinrichtung 152 vorgesehen ist, die dazu eingerichtet ist, den Ozongehalt des aus dem aus der Behandlungskammer 76 ausgelassenen Gasstroms 90 zu reduzieren.

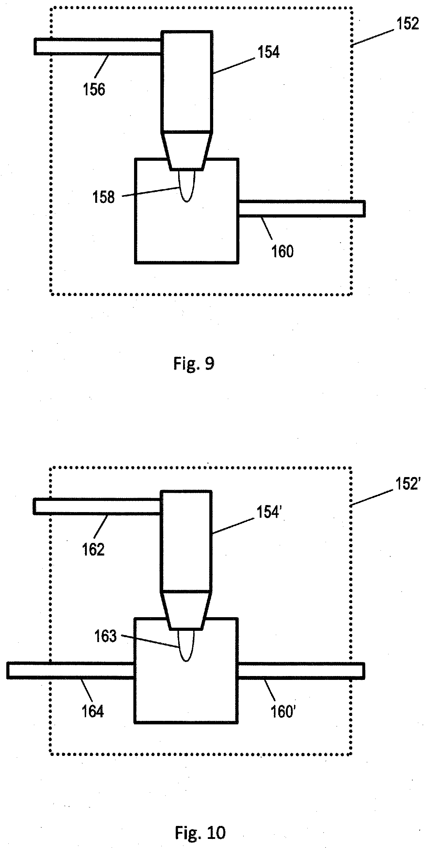

Fig. 9 zeigt die Neutralisationseinrichtung 152 der Vorrichtung 142 aus Fig. 8. Die Neutralisationseinrichtung 152 umfasst eine Plasmadüse 154 zur Erzeugung eines atmosphärischen Plasmastrahls durch hochfrequente Hochspannungsentladungen in einem Arbeitgas. Die Plasmadüse 154 kann insbesondere wie die in Figur 2 dargestellte Plasmadüse 2 ausgebildet sein.

Die Plasmadüse 154 wird über eine Zuleitung 156 mit einem Arbeitsgasstrom versorgt, wobei die Zuleitung 156 an den stromaufwärtigen Teil der Auslassleitung 144 angeschlossen ist, so dass die Plasmadüse 154 mit dem aus der Behandlungskammer 76 ausgelassenen Gasstrom 90 als Arbeitsgasstrom versorgt wird, der dann im Betrieb als Plasmastrahl 158 aus der Plasmadüse 154 austritt. Auf diese Weise wird der Ozongehalt in dem Gasstrom 90 erheblich reduziert. Der als Plasmastrahl 158 aus der Plasmadüse 154 austretende Gasstrom 90 wird dann über eine weitere Leitung 160 dem stromabwärtigen Teil der Auslassleitung 144 zugeführt und gelangt dadurch zur Auslassöffnung 148.

Fig. 10 zeigt eine alternative Neutralisationseinrichtung 152', die anstelle der Neutralisationseinrichtung 152 für die Vorrichtung aus Fig. 8 verwendet werden kann. Die Neutralisationseinrichtung 152' weist ebenfalls eine Plasmadüse 154' auf, die insbesondere wie die Plasmadüse 2 aus Fig. 1 ausgebildet sein kann. Anders als bei der Neutralisationseinrichtung 152 in Fig. 9 wird die Plasmadüse 154' über eine gesonderte Arbeitsgaszuleitung 162 mit Arbeitsgas versorgt, so dass im Betrieb ein Plasmastrahl 163 aus der Plasmadüse 154' austritt. Weiterhin ist eine an dem stromaufwärtigen Teil der Auslassleitung 144 angeschlossene Zuleitung 164 vorgesehen, die den aus der Behandlungskammer 76 ausgelassenen Gasstrom 90 in den Bereich des Plasmastrahls 163 führt, so dass der Gasstrom 90 mit dem Plasmastrahl 163 beaufschlagt wird. Dies führt ebenfalls zu einer Reduzierung des Ozongehalts in dem Gasstrom 90. Der Gasstrom 90 wird dann über die weitere Leitung 160' dem stromabwärtigen Teil der Auslassleitung 144 zugeführt und gelangt dadurch zur Auslassöffnung 148.

Mit der Neutralisationseinrichtung 152 bzw. 152' kann zum Beispiel am Ende eines Desinfektionsvorgangs der noch teilweise mit reaktiven Spezies, insbesondere Ozon, angereicherte Gasstrom 90 aus der Behandlungskammer 76 entfernt, neutralisiert und abgelassen werden, so dass die Ozonbelastung für den Nutzer bei Entnahme der Schutzausrüstungsteile aus der Behandlungskammer 76 und die Umwelt reduziert wird.

Es kann weiterhin eine hinzuschaltbare Frischluftleitung 166 vorgesehen sein, durch die beim Ablassen des Gasstroms 90 aus der Behandlungskammer 76 Frischluft nachströmen kann. Die Frischluftleitung 166 kann beispielweise an die Erzeugungseinheit 88 angeschlossen sein, die am Ende eines Desinfektionsvorgangs insbesondere nicht mehr betrieben wird. Die Frischluftleitung 166 kann auch separat von der Erzeugungseinheit 88 angeordnet sein.

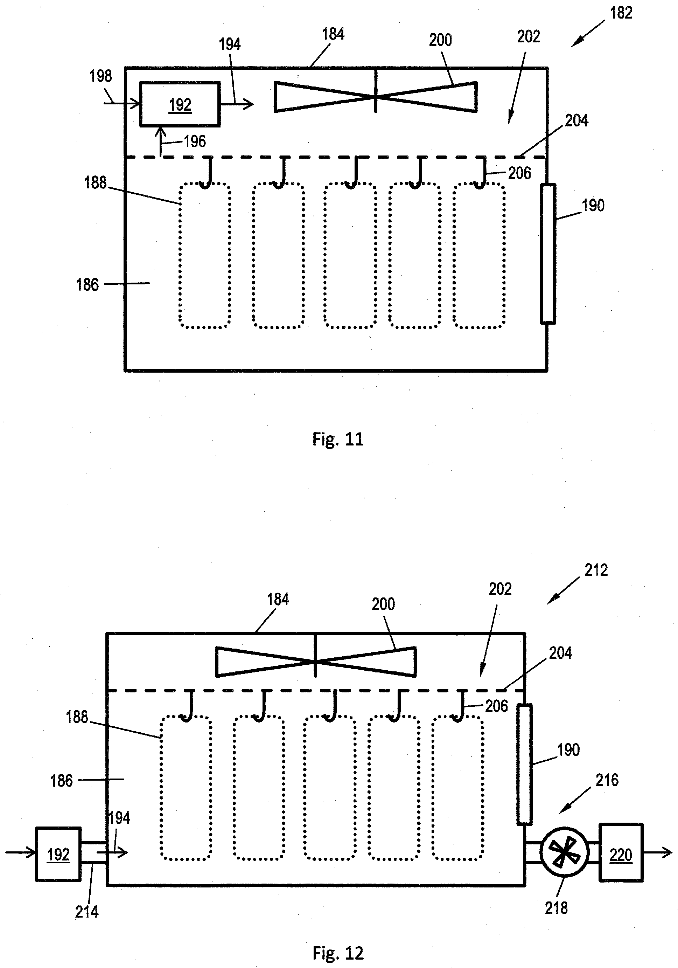

Fig. 11 zeigt ein weiteres Ausführungsbeispiel der Vorrichtung zur Desinfektion von Gegenständen oder Feststoffen, vorzugsweise Schutzausrüstungsteilen, sowie für dessen Verwendung. Die Vorrichtung 182 umfasst ein Gehäuse 184, in der eine Behandlungskammer 186 zur Aufnahme eines oder mehrerer Schutzausrüstungsteile 188 angeordnet ist, eine verschließbare Schleuse 190, zum Beispiel in Form einer verschließbaren Tür oder Klappe, durch die Schutzausrüstungsteile 188 in die Behandlungskammer 186 eingebracht und/oder aus der Behandlungskammer 186 entnommen werden können, und eine Erzeugungseinheit 192 zur Erzeugung reaktiver Spezies in einem Gasstrom, wobei die Erzeugungseinheit 192 Entladungsmittel umfasst, die dazu eingerichtet sind, eine elektrische Entladung in dem Gasstrom zu erzeugen.

Die Erzeugungseinheit 192 ist bei der Vorrichtung 182 in der Behandlungskammer 186 angeordnet. Dadurch gelangt der im Betrieb aus der Erzeugungseinheit 192 austretende und mit reaktiven Spezies angereicherte Gasstrom 194 unmittelbar in die Behandlungskammer 186.

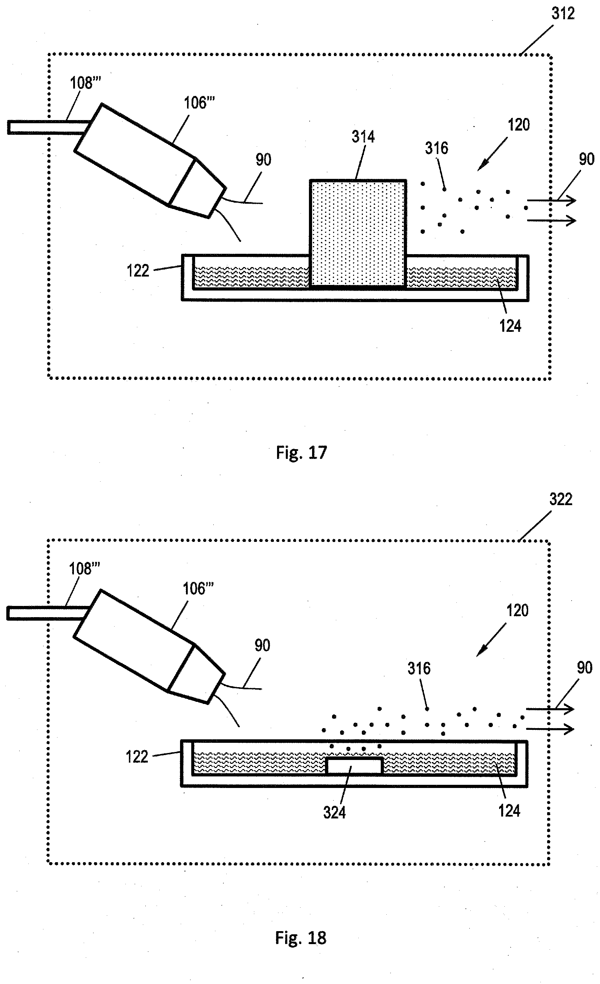

Die Erzeugungseinheit 192 kann wie eine der Erzeugungseinheiten 88, 88', 88" oder 88‴ aus den Fig. 4 - 7 oder wie eine der Erzeugungseinheiten 312 oder 322 aus den Fig. 17 - 18 ausgebildet sein, wobei die Zuleitung 108, 108', 108", 108‴ im Fall der Vorrichtung 182 nicht wie bei der Vorrichtung 72 an ein Umluftsystem angeschlossen ist, sondern an eine Zuleitung 196 aus der Behandlungskammer 186 oder an eine gesonderte Zuleitung 198 für ein Arbeitsgas.

Um den Gasstrom 194 mit den reaktiven Spezies besser innerhalb der Behandlungskammer 186 zu verteilen, ist in der Behandlungskammer 186 vorzugsweise ein Ventilator 200 angeordnet.

In der Behandlungskammer 186 sind weiterhin Positioniervorrichtungen 202 zur Positionierung von Schutzausrüstungsteilen 188 an vorgegebenen Stellen in der Behandlungskammer 186 vorgesehen. Die Positioniervorrichtungen 202 sind vorliegend als Halterung 204 mit Haken 206 zum Aufhängen von Schutzausrüstungsteilen 188 wie Schutzbekleidungsteilen oder Atemschutzmasken ausgebildet.

Durch die Anordnung der Erzeugungseinheit 192 innerhalb der Behandlungskammer 186 kann eine hohe Desinfektionswirkung bewirkt werden.

Fig. 12 zeigt ein weiteres Ausführungsbeispiel der Vorrichtung zur Desinfektion von Gegenständen oder Feststoffen, vorzugsweise Schutzausrüstungsteilen, sowie für dessen Verwendung. Die Vorrichtung 212 weist einen ähnlichen Aufbau auf wie die Vorrichtung 182. Einander entsprechende Komponenten sind mit gleichen Bezugszeichen versehen und es wird auf die obige Beschreibung zu Fig. 11 verwiesen.

Die Vorrichtung 212 unterscheidet sich dadurch von der Vorrichtung 182, dass die Erzeugungseinheit 192 nicht innerhalb, sondern außerhalb der Behandlungskammer 186 angeordnet und über eine Zuleitung 214 an diese angeschlossen ist, so dass der Gasstrom 194 mit den reaktiven Spezies in die Behandlungskammer 186 gelangt.

Weiterhin ist an der Behandlungskammer ein Auslass 216 mit Absaugmitteln in Form eines Ventilators 218 vorgesehen, um den Gasstrom 194 nach Durchlaufen der Behandlungskammer 186 oder am Ende eines Desinfektionsvorgangs aus der Behandlungskammer 186 abzusaugen. An dem Auslass 216 kann auch eine Neutralisationseinrichtung 220 vorgesehen sein, die dazu eingerichtet ist, den Ozongehalt des aus der Behandlungskammer ausgelassenen Gasstroms zu reduzieren. Die Neutralisationseinrichtung 220 kann beispielsweise wie die Neutralisationseinrichtung 152 aus Fig. 9 oder wie die Neutralisationseinrichtung 152' aus Fig. 10 ausgebildet sein.

Durch die Anordnung der Erzeugungseinheit 192 außerhalb der Behandlungskammer 186 kann eine längere Lebensdauer der Erzeugungseinheit 192 erreicht werden.

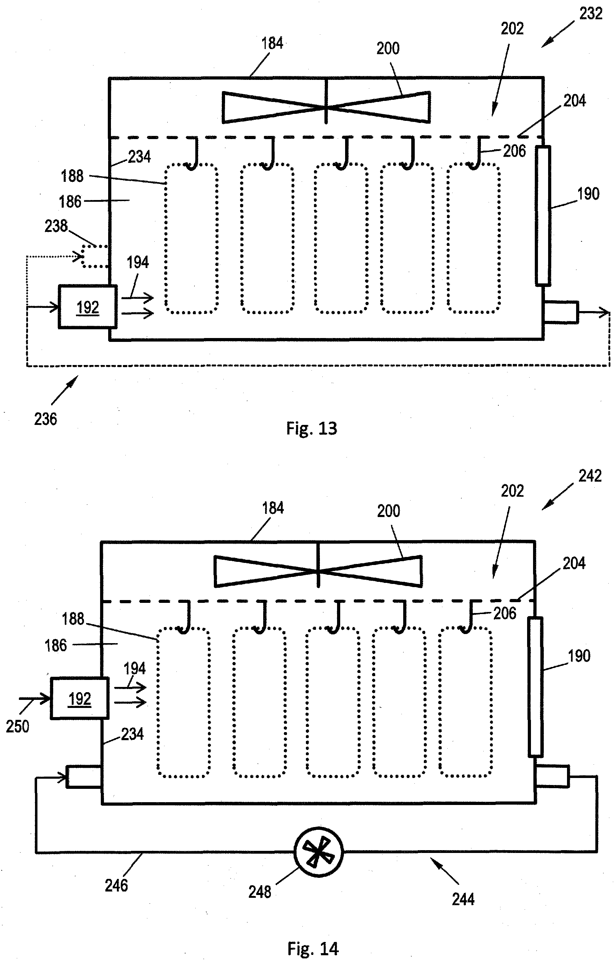

Fig. 13 zeigt ein weiteres Ausführungsbeispiel der Vorrichtung zur Desinfektion von Gegenständen oder Feststoffen, vorzugsweise Schutzausrüstungsteilen, sowie für dessen Verwendung. Die Vorrichtung 232 weist einen ähnlichen Aufbau auf wie die Vorrichtung 182. Einander entsprechende Komponenten sind mit gleichen Bezugszeichen versehen und es wird auf die obige Beschreibung zu Fig. 11 verwiesen.

Die Vorrichtung 232 unterscheidet sich dadurch von der Vorrichtung 182, dass die Erzeugungseinheit 192 in die Wand 234 der Behandlungskammer 186 integriert ist. Auf diese Weise kann eine gute Desinfektionswirkung bei gleichzeitig guter Lebensdauer der Erzeugungseinheit 192 erreicht werden.

Die Vorrichtung 232 kann in einer Variante auch mit einem Umluftsystem 236 ausgebildet werden. Der durch das Umluftsystem 236 geleitete Gasstrom kann vollständig der Erzeugungseinheit 192 zugeführt werden. In einer alternativen Ausgestaltung wird der durch das Umluftsystem 236 geleitete Gasstrom nur teilweise der Erzeugungseinheit 192 zugeführt, während der restliche Gasstrom durch einen parallelen Umluft-Auslass 238 direkt in die Behandlungskammer 186 geleitet wird. Auf diese Weise kann der Durchsatz des Umluftsystems 236 höher gewählt werden als der Maximaldurchsatz der Erzeugungseinheit 192, wodurch eine bessere Verteilung des mit den reaktiven Spezies angereicherten Gasstroms innerhalb der Behandlungskammer 186 erreicht werden kann.

Fig. 14 zeigt ein weiteres Ausführungsbeispiel der Vorrichtung zur Desinfektion von Gegenständen oder Feststoffen, vorzugsweise Schutzausrüstungsteilen, sowie für dessen Verwendung. Die Vorrichtung 242 weist einen ähnlichen Aufbau auf wie die Vorrichtung 182. Einander entsprechende Komponenten sind mit gleichen Bezugszeichen versehen und es wird auf die obige Beschreibung zu Fig. 11 verwiesen.

Die Vorrichtung 242 unterscheidet sich dadurch von der Vorrichtung 182, dass ein Umluftsystem 244 vorgesehen ist, das dazu eingerichtet ist, den Gasstrom aus der Behandlungskammer 186 abzuleiten und der Behandlungskammer 186 über ein Umluftleitungssystem 246 wieder zuzuleiten. Zu diesem Zweck weist das Umluftsystem insbesondere einen Ventilator 248 auf. Die Erzeugungseinheit 192 ist separat vom Umluftsystem ausgebildet und kann wie in Fig. 14 dargestellt in eine Wand 234 der Behandlungskammer 186 integriert sein oder alternativ auch außerhalb der Behandlungskammer 186 angeordnet und über eine Zuleitung mit dieser verbunden sein. In einer weiteren Ausgestaltung kann die Erzeugungseinheit 192 auch innerhalb der Behandlungskammer 186 angeordnet sein.

Die Erzeugungseinheit 192 wird insbesondere über eine separate Arbeitsgasversorgung 250 mit Arbeitsgas versorgt, so dass ein mit reaktiven Spezies angereicherter Gasstrom 194 aus der Erzeugungseinheit 192 austritt. Durch die Trennung von Umluftsystem 244 und Erzeugungseinheit 192 kann die Lebensdauer der Erzeugungseinheit 192 verlängert werden, da die Erzeugungseinheit 192 durch die separate Arbeitsgasversorgung 250 geringerem Verschleiß unterliegt, als wenn ihr ein bereits bzw. noch mit reaktiven Spezies angereicherter Gasstrom als Arbeitsgas zugeführt wird.

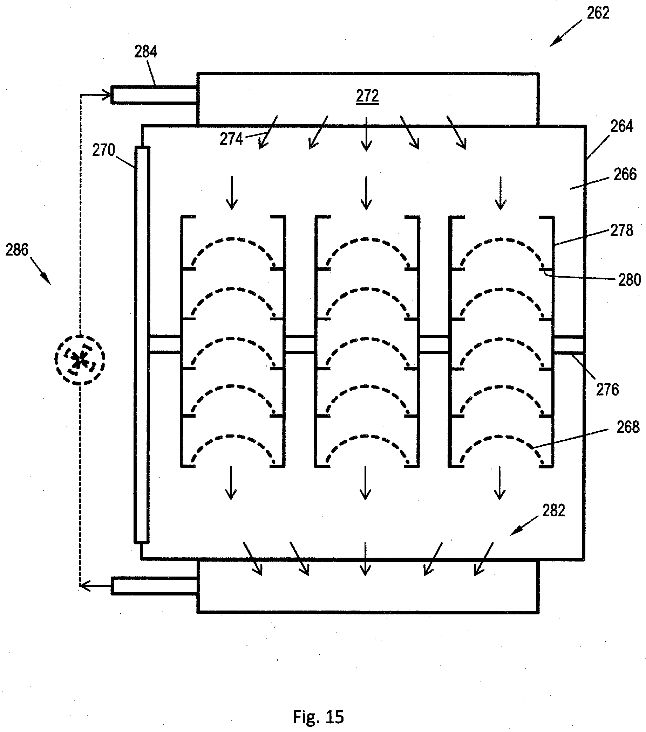

Fig. 15 zeigt ein weiteres Ausführungsbeispiel der Vorrichtung zur Desinfektion von Gegenständen oder Feststoffen, vorzugsweise Schutzausrüstungsteilen, sowie für dessen Verwendung. Die Vorrichtung 262 weist ein Gehäuse 264 mit einer Behandlungskammer 266 zur Aufnahme eines oder mehrerer Schutzausrüstungsteile 268, eine verschließbare Schleuse 270, durch die Schutzausrüstungsteile 268 in die Behandlungskammer 266 eingebracht und/oder aus der Behandlungskammer 266 entnommen werden können, und eine Erzeugungseinheit 272 zur Erzeugung reaktiver Spezies in einem Gasstrom 274 auf, wobei die Erzeugungseinheit derart angeordnet ist, dass der Gasstrom 274 im Betrieb in die Behandlungskammer 266 gelangt. Die Erzeugungseinheit 272 umfasst Entladungsmittel, die dazu eingerichtet sind, eine elektrische Entladung in dem Gasstrom zu erzeugen. Insbesondere kann die Erzeugungseinheit 272 wie eine der Erzeugungseinheiten 88, 88', 88" oder 88‴ aus den Fig. 4 - 7 oder wie eine der Erzeugungseinheiten 312 oder 322 aus den Fig. 17 - 18 ausgebildet sein.

In der Behandlungskammer 266 sind über eine Halterung 276 mehrere rohrförmige Elemente 278 derart angeordnet, dass sie im Betrieb von dem in die Behandlungskammer 266 eingeleiteten Gasstrom 274 durchströmt werden.

Die rohrförmigen Elemente 278 sind jeweils dazu ausgebildet, mehrere Atemschutzmasken 268 derart hintereinander aufzunehmen, dass die Atemschutzmasken 268 beim Durchströmen des rohrförmigen Elements 278 mit dem Gasstrom 274 nacheinander durchströmt werden. Zu diesem Zweck weisen die rohrförmigen Elemente 278 hintereinander angeordnete Halteelemente 280 zum Positionieren der Atemschutzmasken 268 auf. Alternativ zu mehreren Halteelementen 280 für einzelne Atemschutzmasken kann beispielsweise auch ein Halteelement vorgesehen sein, auf dem ein Stapel ineinandergesteckter Atemschutzmasken positioniert wird.

Auf diese Weise wird eine Zwangsdurchströmung der Atemschutzmasken 268 mit dem mit reaktiven Spezies angereicherten Gasstrom 274 und dadurch eine effektive Desinfektion der Atemschutzmasken 268 erreicht.

Die rohrförmigen Elemente 278 stellen Positioniervorrichtungen zur Positionierung von Schutzausrüstungsteilen, nämlich Atemschutzmasken, an vorgegebener Stelle in der Behandlungskammer 266 dar.

Nach dem Durchströmen der Behandlungskammer 266 kann der Gasstrom 274 durch Öffnungen 282 in der Wand der Behandlungskammer 266 aus dieser herausgeleitet werden.

Die Zuleitung 284 der Erzeugungseinheit 272 kann wie bei der Vorrichtung 72 aus Fig. 3 an ein Umluftsystem 286 angeschlossen sein. Alternativ kann die Zuleitung 284 auch als separate Zuleitung für Arbeitsgas ausgebildet sein. In diesem Fall können die Öffnungen 282 wie bei der Vorrichtung 212 aus Fig. 12 Teil eines Auslasses bilden, um den Gasstrom 274 aus der Behandlungskammer 266 auszulassen.

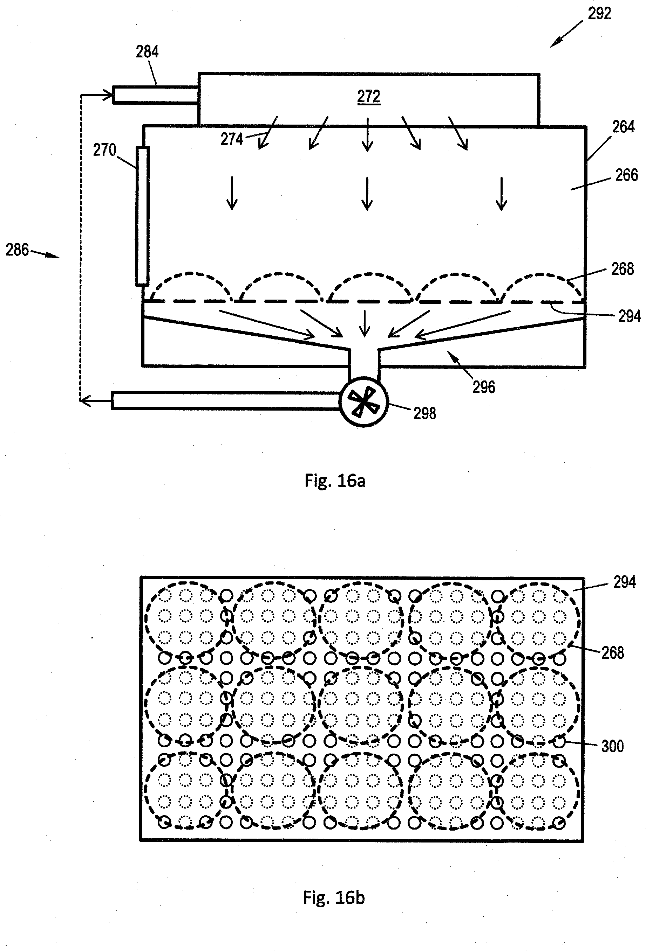

Die Fig. 16a zeigt ein weiteres Ausführungsbeispiel der Vorrichtung zur Desinfektion von Gegenständen oder Feststoffen, vorzugsweise Schutzausrüstungsteilen, sowie für dessen Verwendung. Die Vorrichtung 292 weist einen ähnlichen Aufbau auf wie die Vorrichtung 262 aus Fig. 15, wobei einander entsprechende Komponenten mit gleichen Bezugszeichen versehen sind und insoweit auf die obige Beschreibung zu Fig. 15 Bezug genommen wird.

Die Vorrichtung 292 unterscheidet sich dadurch von der Vorrichtung 262 aus Fig. 15, dass als Positioniervorrichtung ein Lochblech 294 anstelle der rohrförmigen Elemente 278 vorgesehen ist. Unterhalb des Lochblechs 294 ist eine Absaugung 296 mit einem Ventilator 298 vorgesehen, um den Gasstrom 274 durch die Löcher 300 des Lochblechs 294 zu saugen.

Zur Desinfektion von Atemschutzmasken 264 kann der Nutzer diese wie in den Fig. 16a-b auf dem Lochblech 294 anordnen. Fig. 16b zeigt eine Aufsicht auf das Lochblech 294 mit den darauf angeordneten Atemschutzmasken 264.

Im Betrieb wird der von der Erzeugungseinheit 272 mit reaktiven Spezies angereicherte Gasstrom 274 von der Absaugung 296 angesaugt und auf dem Weg zu den Löchern 300 des Lochblechs 294 damit durch das Material der Atemschutzmasken 264 geleitet, so dass diese desinfiziert werden.

Die Vorrichtung 292 weist in Fig. 16a nur ein Lochblech 294 auf. Es ist aber auch denkbar, mehrere Lochbleche übereinander anzuordnen, optional mit jeweiliger Absaugung oder mit einer gemeinsamen Absaugung, so dass eine größere Anzahl von Atemschutzmasken 264 gleichzeitig in der Vorrichtung 292 behandelt werden kann.

Fig. 17 zeigt eine weitere alternative Erzeugungseinheit 312 für die Vorrichtung aus Fig. 3, die anstelle der Erzeugungseinheit 88 für die Vorrichtung aus Fig. 3 verwendet werden kann. Die Erzeugungseinheit 312 weist einen ähnlichen Aufbau auf wie die Erzeugungseinheit 88‴ aus Fig. 7, wobei einander entsprechende Komponenten mit gleichen Bezugszeichen versehen sind und insoweit auf die obige Beschreibung zu Fig. 7 Bezug genommen wird.

Die Erzeugungseinheit 312 unterscheidet sich dadurch von der Erzeugungseinheit 88"', dass in der Wasserwanne 122 ein Verdunstungskörper 314 aus porösem, insbesondere schwammartigem Material angeordnet ist. Der Verdunstungskörper 314 ragt aus dem Wasser 124 hinaus, so dass er von dem Gasstrom 90 umströmt bzw. durchströmt wird.

Der Verdunstungskörper 314 weist relativ zu seinem Volumen eine sehr große Oberfläche auf. Wenn die Wasserwanne 122 mit Wasser 124 gefüllt wird, gelangt dieses auch in den Verdunstungskörper 314 bzw. wird durch Kapillarkräfte in diesen eingesaugt, wodurch eine bessere Verdunstungsleistung erreicht wird. Bei ausreichendem Durchsatz des Gasstroms 90 können von dem Verdunstungskörper 314 zudem Tröpfchen 316 mitgerissen werden, so dass der Gasstrom 90 auch hierdurch befeuchtet wird.

Die Heizmittel 126, die Steuermittel 130 und die Sensoren 128, 134 sowie die Wasserzuleitung 136 sind in Fig. 17 weggelassen, können alternativ aber auch teilweise oder vollständig vorhanden sein.

Fig. 18 zeigt eine weitere alternative Erzeugungseinheit 322 für die Vorrichtung aus Fig. 3, die anstelle der Erzeugungseinheit 88 für die Vorrichtung aus Fig. 3 verwendet werden kann. Die Erzeugungseinheit 322 weist einen ähnlichen Aufbau auf wie die Erzeugungseinheit 88‴ aus Fig. 7, wobei einander entsprechende Komponenten mit gleichen Bezugszeichen versehen sind und insoweit auf die obige Beschreibung zu Fig. 7 Bezug genommen wird.

Die Erzeugungseinheit 322 unterscheidet sich dadurch von der Erzeugungseinheit 88"', dass in der Wasserwanne 122 ein Ultraschallvernebler 324 angeordnet ist. Der Ultraschallvernebler 324 vernebelt im Betrieb das Wasser 124 zu kleinen Tröpfchen 316, die dann vom Gasstrom 90 mitgerissen werden und diesen dadurch befeuchten.

Die Heizmittel 126, die Steuermittel 130 und die Sensoren 128, 134 sowie die Wasserzuleitung 136 sind in Fig. 18 weggelassen, können alternativ aber auch teilweise oder vollständig vorhanden sein.

Anstelle von Wasser 124 kann die Wasserwanne 122 bei der Erzeugungseinheit 88‴, 312 oder 322 auch mit plasmaaktiviertem Wasser befüllt werden, um die Desinfektionswirkung weiter zu verbessern. Die Verwendung eines Verdunstungskörpers 314 oder Verneblers 324 ist in diesem Fall vorteilhaft, da die im plasmaaktivierten Wasser enthaltenden reaktiven Spezies durch die bei diesen Ausgestaltungen bewirkte Bildung von Tröpfchen 316 besser in den Gasstrom 90 gelangen.

Die Vorrichtung und die Verwendung sind zuvor exemplarisch anhand von Ausführungsbeispielen der Vorrichtung zur Desinfektion, insbesondere zur Sterilisation, von Schutzausrüstungsteilen beschrieben worden. Die Vorrichtung kann jedoch auch zur Desinfektion, insbesondere Sterilisation anderer Güter, insbesondere Pulver, Saatgut oder Lebensmittel, insbesondere Gemüse, Obst, Salat, Nüsse wie zum Beispiel Haselnüsse, Mandeln, Hülsenfrüchte oder Gewürze wie zum Beispiel Pfeffer, verwendet werden. Zu diesem Zweck kann die Vorrichtung für die jeweiligen Güter entsprechend dimensioniert und es können zum Beispiel entsprechende Positioniervorrichtungen für die betreffenden Güter vorgesehen werden.

## Patentansprüche

1. Vorrichtung (72, 142, 182, 212, 232, 242, 262, 292) zur Desinfektion, insbesondere zur Sterilisation, von Gegenständen oder Feststoffen, vorzugsweise von Schutzausrüstungsteilen (78, 188, 268), insbesondere von Schutzmasken oder Schutzbekleidung,
- mit einer Behandlungskammer (76, 186, 266) zur Aufnahme eines oder mehrerer Gegenstände oder Feststoffe, vorzugsweise eines oder mehrerer Schutzausrüstungsteile (78, 188, 268),
- mit einer verschließbaren Schleuse (80, 190, 270), durch die Gegenstände oder Feststoffe, vorzugsweise Schutzausrüstungsteile (78, 188, 268), in die Behandlungskammer (76, 186, 266) eingebracht und/oder aus der Behandlungskammer (76, 186, 266) entnommen werden können, und
- mit einer Erzeugungseinheit (88, 88', 88", 88"', 192, 272, 312, 322) zur Erzeugung reaktiver Spezies in einem Gasstrom (23, 38, 90, 114, 116, 194, 274),
- wobei die Erzeugungseinheit (88, 88', 88", 88"', 192, 272, 312, 322) Entladungsmittel (25, 65) umfasst, die dazu eingerichtet sind, eine elektrische Entladung in dem Gasstrom (23, 38, 90, 114, 116, 194, 274) zu erzeugen, und
- wobei die Erzeugungseinheit (88, 88', 88", 88"', 192, 272, 312, 322) derart angeordnet ist, dass der Gasstrom (23, 38, 90, 114, 116, 194, 274) im Betrieb von der Erzeugungseinheit (88, 88', 88", 88"', 192, 272, 312, 322) in die Behandlungskammer (76, 186, 266) gelangt,
**dadurch gekennzeichnet,**
- **dass** die Entladungsmittel (25) zur Erzeugung einer bogenartigen Entladung in einem Gasstrom (23, 38, 90, 114, 116, 194, 274) eingerichtet sind, wobei die bogenartige Entladung durch Anlegen einer hochfrequenten Hochspannung zwischen Elektroden erzeugt wird, und
- **dass** Befeuchtungsmittel (120) vorgesehen sind, die dazu eingerichtet sind, die relative Feuchtigkeit des Gasstroms (23, 38, 90, 114, 116, 194, 274) und/oder die relative Feuchtigkeit in der Behandlungskammer (76, 186, 266) zu erhöhen, vorzugsweise auf eine relative Feuchtigkeit im Bereich von 90 % rF - 100 % rF.

2. Vorrichtung nach Anspruch 1,
**dadurch gekennzeichnet, dass** die Erzeugungseinheit (88", 88‴, 192, 272, 312, 322) eine erste Teilerzeugungseinheit (110) und eine zweite Teilerzeugungseinheit (112) aufweist, wobei die erste Teilerzeugungseinheit (110) erste Entladungsmittel (65) zur Erzeugung einer dielektrisch behinderten Entladung in einem ersten Teilgasstrom (114) umfasst und wobei die zweite Teilerzeugungseinheit (112) zweite Entladungsmittel (25) zur Erzeugung einer hochfrequenten Hochspannungsentladung in einem zweiten Teilgasstrom (116) umfasst.

3. Vorrichtung nach Anspruch 2,
**dadurch gekennzeichnet, dass** die Erzeugungseinheit (88", 88‴, 192, 272, 312, 322) dazu eingerichtet ist, den ersten Teilgasstrom (114) und den zweiten Teilgasstrom (116) zusammenzuführen, insbesondere zu vermischen.

4. Vorrichtung nach einem der Ansprüche 1 bis 3,
**dadurch gekennzeichnet, dass** in der Behandlungskammer (76, 186, 266) ein Ventilator (200) angeordnet ist.

5. Vorrichtung nach einem der Ansprüche 1 bis 4,
**dadurch gekennzeichnet, dass** in der Behandlungskammer (76, 186, 266) ein rohrförmiges Element (278) derart angeordnet ist, dass es im Betrieb von dem in die Behandlungskammer (76, 186, 266) eingeleiteten Gasstrom (23, 38, 90, 114, 116, 194, 274) durchströmt wird, wobei das rohrförmige Element (278) dazu ausgebildet ist, mehrere Gegenstände, vorzugsweise mehrere Schutzausrüstungsteile (78, 188, 268), insbesondere mehrere Atemmasken, derart aufzunehmen, dass die Gegenstände, vorzugsweise die Schutzausrüstungsteile (78, 188, 268), insbesondere Atemschutzmasken, beim Durchströmen des rohrförmigen Elements (278), vorzugsweise nacheinander, mit dem Gasstrom (23, 38, 90, 114, 116, 194, 274) durchströmt werden.

6. Vorrichtung nach einem der Ansprüche 1 bis 5,
**dadurch gekennzeichnet, dass** an der Behandlungskammer (76, 186, 266) ein Auslass (150, 216) vorgesehen ist, um den Gasstrom (23, 38, 90, 114, 116, 194, 274) aus der Behandlungskammer (76, 186, 266) auszulassen, dass an dem Auslass (150, 216) eine Neutralisationseinrichtung (152, 152', 220) vorgesehen ist, die dazu eingerichtet ist, den Ozongehalt des Gasstroms (23, 38, 90, 114, 116, 194, 274) zu reduzieren, und dass die Neutralisationseinrichtung (152, 152', 220) eine Plasmadüse (2, 154, 154') zur Erzeugung eines atmosphärischen Plasmastrahls (26, 158, 158') umfasst.

7. Vorrichtung nach Anspruch 6,
**dadurch gekennzeichnet, dass** die Plasmadüse (2, 154, 154') dazu eingerichtet ist, einen Plasmastrahl (26, 158, 158') durch hochfrequente Hochspannungsentladungen in einem Arbeitsgasstrom zu erzeugen.

8. Vorrichtung nach Anspruch 6 oder 7,
**dadurch gekennzeichnet, dass** der Gasstrom (23, 38, 90, 114, 116, 194, 274) als Arbeitsgasstrom durch die Plasmadüse (2, 154) geleitet wird.

9. Vorrichtung nach Anspruch 6 oder 7,
**dadurch gekennzeichnet, dass** der Gasstrom (23, 38, 90, 114, 116, 194, 274) mit dem aus der Plasmadüse (2, 154') austretenden Plasmastrahl (26, 158') beaufschlagt wird.

10. Vorrichtung nach einem der Ansprüche 1 bis 9,
**dadurch gekennzeichnet, dass** ein Umluftsystem (94, 236, 244, 286) vorgesehen ist, das dazu eingerichtet ist, den Gasstrom (23, 38, 90, 114, 116, 194, 274) aus der Behandlungskammer (76, 186, 266) abzuleiten und der Behandlungskammer (76, 186, 266) über ein Umluftleitungssystem (98, 246) wieder zuzuleiten, dass das Umluftsystem (94, 236, 244, 286) einen Ventilator (100, 248), insbesondere einen Seitenkanalverdichter, aufweist, der dazu eingerichtet ist, den Gasstrom (23, 38, 90, 114, 116, 194, 274) aus der Behandlungskammer (76, 186, 266) abzusaugen und durch das Umluftleitungssystem (98, 246) zu leiten, und dass die Erzeugungseinheit (88, 88', 88", 88"', 192, 272, 312, 322) derart in das Umluftsystem (94, 236, 244, 286) integriert ist, dass der durch das Umluftsystem (94, 236, 244, 286) geleitete Gasstrom (23, 38, 90, 114, 116, 194, 274) zumindest teilweise der Erzeugungseinheit (88, 88', 88", 88"', 192, 272, 312, 322) zugeführt wird.

11. Vorrichtung nach einem der Ansprüche 1 bis 10,
**dadurch gekennzeichnet, dass** die Befeuchtungsmittel (120) dazu eingerichtet sind, die relative Feuchtigkeit des Gasstroms (23, 38, 90, 114, 116, 194, 274) und/oder die relative Feuchtigkeit in der Behandlungskammer (76, 186, 266) auf eine relative Feuchtigkeit im Bereich von 95 % rF - 99 % rF zu erhöhen.

12. Vorrichtung nach einem der Ansprüche 1 bis 11,
**dadurch gekennzeichnet, dass** die Befeuchtungsmittel (120) eine vorzugsweise beheizbare Wasserwanne (122) umfassen.

13. Vorrichtung nach Anspruch 12,
**dadurch gekennzeichnet, dass** die Wasserwanne (122) derart angeordnet ist, dass der Gasstrom (23, 38, 90, 114, 116, 194, 274) von der Erzeugungseinheit (88, 88', 88", 88"', 192, 272, 312, 322) im Betrieb auf die Wasserwanne (122) gerichtet wird.

14. Vorrichtung nach Anspruch 12 oder 13,
**dadurch gekennzeichnet, dass** in der Wasserwanne ein Verdunstungskörper (314) und/oder ein Vernebler (324) angeordnet ist.

15. Verwendung der Vorrichtung (72, 142, 182, 212, 232, 242, 262, 292) nach einem der Ansprüche 1 bis 14 zur Desinfektion, insbesondere zur Sterilisation, von Gegenständen oder Feststoffen, vorzugsweise von Schutzausrüstungsteilen (78, 188, 268), insbesondere von Schutzmasken oder Schutzbekleidung.

## Claims

1. Apparatus (72, 142, 182, 212, 232, 242, 262, 292) for disinfecting, in particular for sterilizing, objects or solids, preferably pieces of protective equipment (78, 188, 268), in particular protective masks or protective clothing,
- comprising a treatment chamber (76, 186, 266) for receiving one or more objects or solids, preferably one or more pieces of protective equipment (78, 188, 268),
- comprising a closable airlock (80, 190, 270), through which objects or solids, preferably pieces of protective equipment (78, 188, 268), can be introduced into the treatment chamber (76, 186, 266) and/or removed from the treatment chamber (76, 186, 266), and
- comprising a generation unit (88, 88', 88", 88‴, 192, 272, 312, 322) for generating reactive species in a gas stream (23, 38, 90, 114, 116, 194, 274),
- wherein the generation unit (88, 88', 88", 88‴, 192, 272, 312, 322) comprises discharge means (25, 65), which are configured to generate an electrical discharge in the gas stream (23, 38, 90, 114, 116, 194, 274), and
- wherein the generation unit (88, 88', 88", 88‴, 192, 272, 312, 322) is arranged such that, during operation, the gas stream (23, 38, 90, 114, 116, 194, 274) passes from the generation unit (88, 88', 88", 88‴, 192, 272, 312, 322) into the treatment chamber (76, 186, 266),
**characterized**
- **in that** the discharge means (25) are configured to generate an arc-like discharge in a gas stream (23, 38, 90, 114, 116, 194, 274), wherein the arc-like discharge is generated by applying a high-frequency high voltage between electrodes, and
- **in that** humidifying means (120) are provided, which are configured to increase the relative humidity of the gas stream (23, 38, 90, 114, 116, 194, 274) and/or the relative humidity in the treatment chamber (76, 186, 266), preferably to a relative humidity in the range of 90% RH to 100% RH.

2. The device according to claim 1,
**characterized in that** the generation unit (88", 88‴, 192, 272, 312, 322) comprises a first part generation unit (110) and a second part generation unit (112), wherein the first part generation unit (110) comprises first discharge means (65) for generating a dielectric barrier discharge in a first partial gas stream (114), and wherein the second part generation unit (112) comprises second discharge means (25) for generating a high-frequency high-voltage discharge in a second partial gas stream (116).

3. The device according to claim 2,
**characterized in that** the generation unit (88", 88‴, 192, 272, 312, 322) is configured to combine, in particular to mix, the first partial gas stream (114) and the second partial gas stream (116).

4. The device according to any one of claims 1 to 3,
**characterized in that** a fan (200) is arranged in the treatment chamber (76, 186, 266).

5. The device according to any one of claims 1 to 4,
**characterized in that** a tubular element (278) is arranged in the treatment chamber (76, 186, 266) in such a way that, during operation, the gas stream (23, 38, 90, 114, 116, 194, 274) introduced into the treatment chamber (76, 186, 266) flows through it, wherein the tubular element (278) is configured to receive a plurality of objects, preferably a plurality of pieces of protective equipment (78, 188, 268), in particular a plurality of breathing masks, in such a manner that the objects, preferably the pieces of protective equipment (78, 188, 268), in particular breathing masks, are flowed through by the gas stream (23, 38, 90, 114, 116, 194, 274), preferably one after the other, when the gas stream flows through the tubular element (278).

6. The device according to any one of claims 1 to 5,
**characterized in that** an outlet (150, 216) is provided on the treatment chamber (76, 186, 266) for discharging the gas stream (23, 38, 90, 114, 116, 194, 274) from the treatment chamber (76, 186, 266), **in that** a neutralization device (152, 152', 220) is provided at the outlet (150, 216), which neutralization device is configured to reduce the ozone content of the gas stream (23, 38, 90, 114, 116, 194, 274), and **in that** the neutralization device (152, 152', 220) comprises a plasma nozzle (2, 154, 154') for generating an atmospheric plasma jet (26, 158, 158').

7. The device according to claim 6,
**characterized in that** the plasma nozzle (2, 154, 154') is configured to generate a plasma jet (26, 158, 158') by high-frequency high-voltage discharges in a working gas stream.

8. The device according to claim 6 or 7,
**characterized in that** the gas stream (23, 38, 90, 114, 116, 194, 274) is passed through the plasma nozzle (2, 154) as a working gas stream.

9. The device according to claim 6 or 7,
**characterized in that** the gas stream (23, 38, 90, 114, 116, 194, 274) is impinged with the plasma jet (26, 158') emerging from the plasma nozzle (2, 154').

10. The device according to any one of claims 1 to 9,
**characterized in that** a recirculation system (94, 236, 244, 286) is provided, which is configured to discharge the gas stream (23, 38, 90, 114, 116, 194, 274) from the treatment chamber (76, 186, 266) and to resupply it to the treatment chamber (76, 186, 266) via a recirculation duct system (98, 246), **in that** the recirculation system (94, 236, 244, 286) comprises a fan (100, 248), in particular a side channel compressor, which fan is configured to extract the gas stream (23, 38, 90, 114, 116, 194, 274) from the treatment chamber (76, 186, 266) and to direct it through the recirculation duct system (98, 246), and **in that** the generation unit (88, 88', 88", 88‴, 192, 272, 312, 322) is integrated into the recirculation system (94, 236, 244, 286) in such a manner that the gas stream (23, 38, 90, 114, 116, 194, 274) conducted through the recirculation system (94, 236, 244, 286) is at least partially supplied to the generation unit (88, 88', 88", 88‴, 192, 272, 312, 322).

11. The device according to any one of claims 1 to 10,
**characterized in that** the humidifying means (120) are configured to increase the relative humidity of the gas stream (23, 38, 90, 114, 116, 194, 274) and/or the relative humidity in the treatment chamber (76, 186, 266) to a relative humidity in the range of 95% RH to 99% RH.

12. The device according to any one of claims 1 to 11,
**characterized in that** the humidifying means (120) comprise a preferably heatable water trough (122).

13. The device according to claim 12,
**characterized in that** the water trough (122) is arranged such that, during operation, the gas stream (23, 38, 90, 114, 116, 194, 274) from the generation unit (88, 88', 88", 88‴, 192, 272, 312, 322) is directed onto the water trough (122).

14. The device according to claim 12 or 13,
**characterized in that** an evaporation body (314) and/or a nebulizer (324) is arranged in the water trough.

15. Use of the apparatus (72, 142, 182, 212, 232, 242, 262, 292) according to any one of claims 1 to 14 for disinfecting, in particular for sterilizing, objects or solids, preferably pieces of protective equipment (78, 188, 268), in particular protective masks or protective clothing.

## Revendications

1. Dispositif (72, 142, 182, 212, 232, 242, 262, 292) de désinfection, en particulier de stérilisation, d'objets ou de matières solides, de préférence d'éléments de protection (78, 188, 268), en particulier de masques de protection ou de vêtements de protection,
- avec une chambre de traitement (76, 186, 266) pour accueillir un ou plusieurs objets ou une ou plusieurs matières solides, de préférence un ou plusieurs éléments de protection (78, 188, 268),
- avec un sas refermable (80, 190, 270) à travers lequel on peut introduire des objets ou des matières solides, de préférence des éléments de protection (78, 188, 268) dans la chambre de traitement (76, 186, 266) et/ou les extraire hors de la chambre de traitement (76, 186, 266), et
- avec une unité de production (88, 88', 88", 88‴, 192, 272, 312, 322) pour produire des espèces réactives dans un flux gazeux (23, 38, 90, 114, 116, 194, 274),
- dans lequel l'unité de production (88, 88', 88", 88‴, 192, 272, 312, 322) comprend des moyens de décharge (25, 65) conçus pour produire une décharge électrique dans le flux gazeux (23, 38, 90, 114, 116, 194, 274), et
- dans lequel l'unité de production (88, 88', 88", 88‴, 192, 272, 312, 322) est conçu de telle sorte que le flux gazeux (23, 38, 90, 114, 116, 194, 274) pendant le fonctionnement de l'unité de production (88, 88', 88", 88‴, 192, 272, 312, 322) parvienne dans la chambre de traitement (76, 186, 266), **caractérisé en ce que**
- les moyens de décharge (25) sont conçus pour produire un arc de décharge dans un flux gazeux (23, 38, 90, 114, 116, 194, 274), l'arc de décharge étant produit par création d'une haute tension à haute fréquence entre des électrodes, et
- des outils d'humidification (120) sont prévus, qui sont conçus pour augmenter l'humidité relative du flux gazeux (23, 38, 90, 114, 116, 194, 274) et/ou l'humidité relative dans la chambre de traitement (76, 186, 266), de préférence à une humidité relative dans la gamme de 90 % HR à 100 % HR.

2. Dispositif selon la revendication 1,
**caractérisé en ce que** l'unité de production (88", 88‴, 192, 272, 312, 322) comprend une première unité de production partielle (110) et une seconde unité de production partielle (112), la première unité de production partielle (110) comprenant de premières unités de décharge (65) pour produire une décharge diélectrique interdite dans un premier flux gazeux partiel (114) et la seconde unité de production partielle (112) comprenant de secondes unités de décharge (25) pour produire une décharge haute tension haute fréquence dans un second flux gazeux partiel (116).

3. Dispositif selon la revendication 2,
**caractérisé en ce que** l'unité de production (88", 88‴, 192, 272, 312, 322) est conçue pour réunir le premier flux gazeux partiel (114) et le second flux gazeux partiel (116), en particulier pour les mélanger.

4. Dispositif selon l'une des revendications 1 à 3,
**caractérisé en ce qu'**un ventilateur (200) est disposé dans la chambre de traitement (76, 186, 266).

5. Dispositif selon l'une des revendications 1 à 4,
**caractérisé en ce que** dans la chambre de traitement (76, 186, 266) est disposé un élément tubulaire (278) de telle sorte qu'en fonctionnement, il soit traversé par le flux gazeux (23, 38, 90, 114, 116, 194, 274) introduit dans la chambre de traitement (76, 186, 266), l'élément tubulaire (278) étant conçu pour accueillir plusieurs objets, de préférence plusieurs éléments de protection (78, 188, 268), en particulier plusieurs masques de protection, de sorte que les objets, de préférence les éléments de protection (78, 188, 268), en particulier des masques de protection, soient traversés par le flux gazeux (23, 38, 90, 114, 116, 194, 274) lors de la traversée de l'élément tubulaire (278) par le flux gazeux, de préférence les uns derrière les autres.

6. Dispositif selon l'une des revendications 1 à 5,
**caractérisé en ce qu'**est prévue une sortie (150, 216) sur la chambre de traitement (76, 186, 266) pour évacuer le flux gazeux (23, 38, 90, 114, 116, 194, 274) de la chambre de traitement (76, 186, 266), **en ce qu'**est prévue au niveau de la sortie (150, 216) un dispositif de neutralisation (152, 152', 220) conçu pour réduire la teneur en ozone du flux gazeux (23, 38, 90, 114, 116, 194, 274), et **en ce que** le dispositif de neutralisation (152, 152', 220) comprend une buse à plasma (2, 154, 154') pour produire un faisceau de plasma atmosphérique (26, 158, 158').

7. Dispositif selon la revendication 6,
**caractérisé en ce que** la buse à plasma (2, 154, 154') est conçue pour produire un faisceau de plasma (26, 158, 158') par décharges haute tension haute fréquence dans un flux de gaz de travail.

8. Dispositif selon la revendication 6 ou 7,
**caractérisé en ce que** le flux gazeux (23, 38, 90, 114, 116, 194, 274) est guidé à travers la buse de plasma (2, 154) en tant que flux de gaz de travail.

9. Dispositif selon la revendication 6 ou 7,
**caractérisé en ce que** le flux gazeux (23, 38, 90, 114, 116, 194, 274) est alimenté par le faisceau de plasma (26, 158') issu de la buse de plasma (2, 154').

10. Dispositif selon l'une des revendications 1 à 9,
**caractérisé en ce qu'**est prévu un système de circulation de gaz (94, 236, 244, 286) conçu pour évacuer le flux gazeux (23, 38, 90, 114, 116, 194, 274) de la chambre de traitement (76, 186, 266) et de réalimenter la chambre de traitement (76, 186, 266) via un système de guidage de circulation de gaz (98, 246), **en ce que** le système de circulation de gaz (94, 236, 244, 286) comporte un ventilateur (100, 248), en particulier un compresseur à canal latéral, conçu pour aspirer le flux gazeux (23, 38, 90, 114, 116, 194, 274) hors de la chambre de traitement (76, 186, 266) et pour le conduire à travers le système de guidage de circulation de gaz (98, 246), et **en ce que** l'unité de production (88, 88', 88", 88‴, 192, 272, 312, 322) est intégré dans le système de circulation de gaz (94, 236, 244, 286), de sorte que le flux gazeux (23, 38, 90, 114, 116, 194, 274) guidé à travers le système de guidage de circulation de gaz (94, 236, 244, 286) est acheminé au moins partiellement à l'unité de production (88, 88', 88", 88‴, 192, 272, 312, 322).

11. Dispositif selon l'une des revendications 1 à 10,
**caractérisé en ce que** les outils d'humidification (120) sont conçus pour augmenter l'humidité relative du flux gazeux (23, 38, 90, 114, 116, 194, 274)
et/ou l'humidité relative dans la chambre de traitement (76, 186, 266) à hauteur d'une valeur d'humidité relative dans la gamme de 95 % HR à 99 % HR.

12. Dispositif selon l'une des revendications 1 à 11,
**caractérisé en ce que** les outils d'humidification (120) comprennent une cuve d'eau (122) de préférence chauffable.

13. Dispositif selon la revendication 12,
**caractérisé en ce que** la cuve d'eau (122) est conçue de telle sorte que le flux gazeux (23, 38, 90, 114, 116, 194, 274) soit dirigé en fonctionnement de l'unité de production (88, 88', 88", 88‴, 192, 272, 312, 322) vers la cuve d'eau (122).

14. Dispositif selon la revendication 12 ou 13,
**caractérisé en ce qu'**un vaporisateur (314) et/ou un nébuliseur (324) est disposé dans la cuve d'eau.

15. Utilisation du dispositif (72, 142, 182, 212, 232, 242, 262, 292) selon l'une des revendications 1 à 14 pour la désinfection, en particulier pour la stérilisation, d'objets ou de matières solides, de préférence d'éléments de protection (78, 188, 268), en particulier de masques de protection ou de vêtements de protection.
